(19) Europäisches Patentamt
European Patent Office
Office européen des brevets

(11) Numéro de publication: **0 119 892**
**B1**

(12) # FASCICULE DE BREVET EUROPÉEN

(45) Date de publication du fascicule du brevet: **13.08.86**

(51) Int. Cl.⁴: **C 07 C 83/00**, C 07 C 121/75, C 07 D 215/20, A 01 N 33/24

(21) Numéro de dépôt: **84400346.7**

(22) Date de dépôt: **20.02.84**

(54) **Nouveau dérivés de l'hydroxylamine, leur procédé de préparation, leur application comme facteur de croissance des végétaux et les compositions les renfermant.**

(30) Priorité: **23.02.83 FR 8302916**

(43) Date de publication de la demande: **26.09.84 Bulletin 84/39**

(45) Mention de la délivrance du brevet: **13.08.86 Bulletin 86/33**

(84) Etats contractants désignés: **AT BE CH DE FR GB IT LI LU NL SE**

(56) Documents cité: **JOURNAL OF MEDICINAL CHEMISTRY, vol. 10, no. 3, mai 1967, page 512, Washington, D.C., USA, V.J. BAUER et al.: "The synthesis of aryloxycarbamates"**

(73) Titulaire: **ROUSSEL- UCLAF, 35, boulevard des Invalides, F-75007 Paris (FR)**

(72) Inventeur: **Tessier, Jean, 30, rue Jean Moulin, F-94300 Vincennes (FR)**
Inventeur: **Girault, Pierre, 4, rue des Abbesses, F-75018 Paris (FR)**
Inventeur: **Herve, Jean- Jacques, 44, Parc du Pin Vert Chemin du Pin Vert, F-13400 Aubagne (FR)**
Inventeur: **Van Assche, Charles Jacques, Super Bouvière B. 7 83, Boulevard du Redon, F-13009 Marseille (FR)**

(74) Mandataire: **Fritel, Hubert, Département des Brevets ROUSSEL UCLAF B.P. no 9, F-93230 Romainville (FR)**

EP 0 119 892 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Déscription**

L'invention concerne de nouveaux dérivés de l'hydroxylamine, leur procédé de préparation, leur application comme facteurs de croissance des végétaux et les compositions les renfermant.

L'invention a pour objet les composés de formule (I)

$$Ar—O—NH_2 \qquad (I)$$

dans laquelle Ar représente un radical aromatique ou hétéroaromatique, mono ou polycyclique, éventuellement substitué par un ou plusieurs radicaux choisis dans le groupe constitué par le radical OH, les atomes d'halogène, les groupements

$NO_2$ et CN, les radicaux

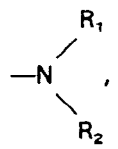 , 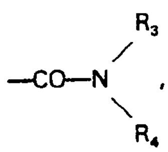 , 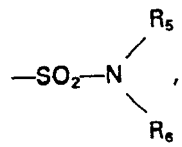 ,

dans lesquels $R_1$, $R_2$, $R_3$, $R_4$, $R_5$ et $R_6$, identiques ou différents, représentent un atome d'hydrogène ou un radical alcoyle linéaire ou ramifié renfermant de 1 à 8 atomes de carbone; les radicaux $R_7$ et $OR_8$, $R_7$ et $R_8$ représentant des radicaux alcoyles linéaires ou ramifiés, saturés ou insaturés renferment jusqu'à 8 atomes de carbone, éventuellement substitués par un ou plusieurs atomes d'halogène ou par un radical cyano, les radicaux —CO $R_9$, les radicaux

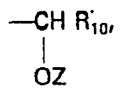

Z représentant un atome d'hydrogène, un radical alcoyle, linéaire ou ramifié, saturé ou insaturé, renfermant jusque'à 8 atomes de carbone ou un radical acyle renfermant de 2 à 18 atomes de carbone, les radicaux $NHCOR_{11}$, $SO_2$ $R_{12}$, $SO_3$ $R_{13}$ dans lesquels $R_9$, $R_{10}$, $R_{11}$, $R_{12}$ et $R_{13}$ représentant un radical alcoyle renfermant de 1 à 8 atomes de carbone, les radicaux $CO_2$ $R_{14}$ dans lesquels $R_{14}$ représente un atome d'hydrogène ou un radical alcoyle renfermant de 1 à 8 atomes de carbone, les radicaux aryle renfermant de 6 à 14 atomes de carbone, les groupements $—CH_2—CN$ ou $—CH_2\text{-}SO_2—R_{15}$ dans lesquels $R_{15}$ représente un radical alcoyle renfermant de 1 à 8 atomes de carbone ou un radical aryle renferment de 6 à 14 atomes de carbone, éventuellement substitué par un radical alcoyle renfermant de 1 à 8 atomes de carbone et les groupements $—OR_{16}$ dans lesquels $R_{16}$ représente un radical aryle renfermant de 6 à 14 atomes de carbone éventuellement substitué par un radical alcoyle renfermant de 1 à 8 atomes de carbone, par ~~un~~ atome d'halogène ou par un radical $NO_2$, les substituants de Ar pouvant éventuellement former des cycles renfermant éventuellement un ou plusieurs hétéroatomes choisis parmi l'oxygène, l'azote et le soufre, ainsi ne représente ni un radical phényle non substitué, ni un radical phényle monosubstitué par un radical méthyle en 2, 3 ou 4, par un radical nitro en 2 ou 4, par un atome de chlore en 3 ou 4, par un atome de brome en 4 ou par un radical $CF_3$ en 3 ou 4, ni un radical phényle ou bien disubstitué en 2 par un radical nitro et en 4 par un radical $CF_3$ ou par un radical nitro ou bien disubstitué en 2 et 6 par un radical nitro, ni un radical phényle trisubstitué par des radicaux nitro en 2, 4 et 6 ou par des radicaux nitro en 2 et 4 et par un radical $CF_3$ en 6.

Lorsque Ar représente un radical aromatique, il s'agit de préférence du radical phényle, naphtyle ou anthracényle.

Lorsque Ar représente un radical hétéroaromatique, il s'agit de préférence du radical pyridyle, quinoléyle, benzothiazolyle ou oxazolyle.

Lorsque Ar est substitué par un halogène, on entend de préférence par halogène le fluor, le chlore ou le brome.

Lorsque $R_1$, $R_2$, $R_3$, $R_4$, $R_5$ ou $R_6$ représente un radical alcoyle, il s'agit de préférence du radical méthyle, éthyle, n-propyle ou n-butyle.

Lorsque $R_7$, $R_8$, $R_9$, $R_{10}$, $R_{11}$, $R_{12}$, $R_{13}$ ou $R_{14}$ représente un radical alcoyle, il s'agit de préférence d'un radical méthyle, éthyle, n-propyle, isopropyle, n-butyle, isobutyle ou terbutyle.

Lorsque $R_7$ ou $R_8$ représente un radical alcoyle substitué par ou plusieurs atomes d'halogène, il s'agit de préférence des radicaux $CF_3$, $CHCl_2$, C $CL_3$, $OCF_3$, $OCHCl_2$ ou $OCCl_3$.

Lorsque $R_7$ ou $R_8$ représente un radical alcoyle insaturé il s'agit de préférence du radical vinyle, allyle ou propargyle.

Lorsque Z représente un radical alcoyle saturé, il s'agit de préférence du radical méthyle, éthyle, n-propyle, isopropyle ou terbutyle.

Lorsque Z représente un radical alcoyle insaturé, il s'agit de préférence du radical vinyle, allyle ou propargyle.

Lorsque Z représente un radical acyle, il s'agit de préférence du radical acétyloxy ou propionyloxy.

Lorsque Ar est substitué par un radical aryle, il s'agit de préférence d'un radical aryle substitué par un radical phényle.

Lorsque $R_{15}$ représente un radical alcoyle, il s'agit de préférence d'un radical méthyle, éthyle, n-propyle ou n-butyle.

Lorsque $R_{15}$ représente un radical aryle, il s'agit de préférence d'un radical phényle ou paratolyle.

$R_{16}$ représente un radical aryle à savoir, de préférence, phényle et lorsque $R_{16}$ est substitué, le substituant est, de préférence, un radical méthyle, éthyle, n-propyle, isopropyle, un atome de chlore, de brome ou d'iode ou un radical nitro.

Lorsque Ar représente un radical aromatique ou hétéroaromatique substitué par plusieurs substituants formant des cycles entre eux, il s'agit de préférence des radicaux:

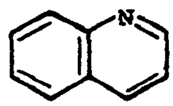 , 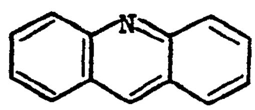 , 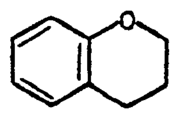 , 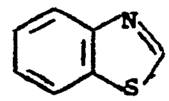 ,

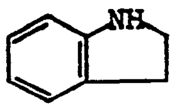 ou 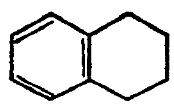

Comme sels des produits de formule (I), on peut citer les sels d'addition avec les acides forts tels que les acides chlorhydrique, bromhydrique, sulfurique, nitrique ou trifluoroacétique.

L'invention a tout particulièrement pour objet les composés de formule (I), pour lesquels Ar représente un radical phényle substitué par un ou plusieurs des radicaux indiqués précédemment, ainsi que leurs sels d'addition avec les acides.

Parmi ces derniers composés, on peut citer les composés de formule (I), dans lesquels l'un au moins des substituants du radical phényle est un radical nitro et les composés de formule (I), dans lesquels l'un au moins des substituants du radical phényle est un atome de chlore en para.

Comme composés préférés de l'invention on peut citer par exemple less composés pour lesquels Ar représente un radical phényle substitué en 2 par un radical nitro et en 4 par un atome de chlore, ceux pour lesquels Ar représente un radical phényle substitué en 4 par un radical nitro et en 3 par un radical alcoxyle renfermant de 1 à 8 atomes de carbone, notamment par un radical méthoxy ou éthoxy; on peut citer également les composés de formule (I) pour lesquels Ar représente un radical phényle substitué en 4 par un radical nitro et en 2 par un radical alcoyle linéaire ou ramifié renfermant de 1 à 8 atomes de carbone, notamment par un radical méthyle ou éthyle.

Comme composés préférés de l'invention, on peut encore citer les composés de formule (I) pour lesquels Ar représente le radical:

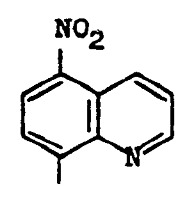

éventuellement substitué par un our plusieurs des substituants mentionnés précédemment, ainsi que ceux pour lesquels Ar représente le radical:

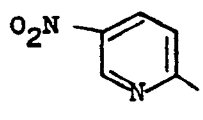

L'invention a tout spécialement pour objet les produits décrits dans les exemples et notamment l'O (2-methyl 4-nitro phényl) hydroxylamine, l'O (4-nitro 3-méthoxy phényl) hydroxylamine ou encore:

— l'O-(5-nitro 8-quinoléyl) hydroxylamine,
— l'O-(2-chloro 4-nitro phényl) hydroxylamine,
— l'O-(2-nitro 4-chloro phényl) hydroxylamine,
— l'O-(2,4-dichloro phényl)hydroxylamine,
— l'O-(4-nitro 2-trifluorométhyl phényl) hydroxylamine,
— l'O-/2(1RS hydroxy éthyl) 4-nitro phényl/ hydroxylamine,

---

— l'O-(4-cyano phényl) hydroxylamine,
— l'O-/3(propyn 2-yloxy) 4-nitro phényl/ hydroxylamine,
— l'O-(3,4-dichlorophényl) hydroxylamine,

ainsi que leurs sels d'addition avec les acides.

L'invention a également en particulier pour objet les composés dont les structures chimiques sont indiquées ci-après:

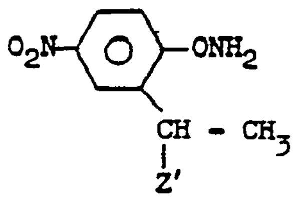

avec Z' représentant un radical acyloxy renfermant jusque'à 8 atomes de carbone ou représentant un atome de fluor, de chlore ou de brome.

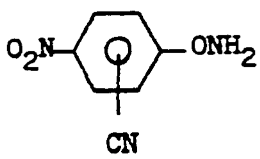

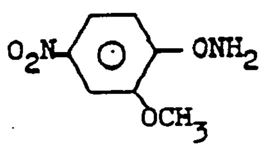

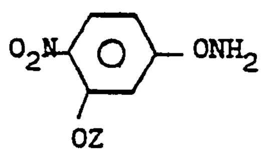

avec Z représentant:

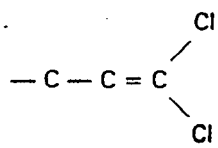

— $CH_2$ —◁

— $CH_2$ — CN

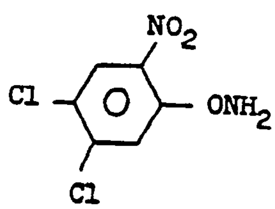

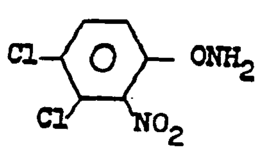

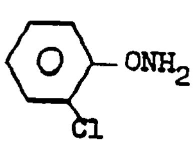

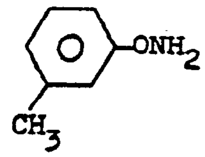

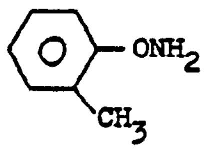

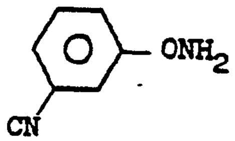

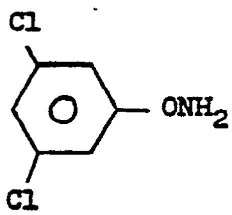

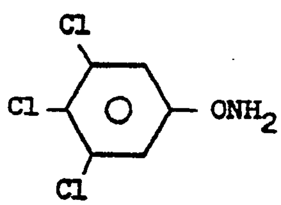

Les composés de formule (I) peuvent être utilisés en agriculture comme facteurs de croissance des végétaux, pour améliorer l'état physiologique des végétaux cultivés et obtenir une augmentation du poids des récoltes. On peut, par exemple, obtenir une augmentation du poids des récoltes des racines, des gousses, des fruits et des feuilles. On peut utiliser les composés de formule (I) sur le soja, le blé, l'orge, l'avoine, le coton, le haricot, la tomate, l'oignon, la pomme de terre, les salades, les rosacées, les composacées, etc. ... et plus spécifiquement, sur les plantes dites en $C_3$ (c'est-à-dire, les plantes dont le premier produit formé au cours de la photosynthèse est une molécule à trois atomes de carbone).

Les composés de formule (I) ont pour effet de diminuer l'inhibition par l'oxygène de la photosynthèse.

L'invention a donc pour objet les composés de formule (I), tels que définis précédemment, ainsi que leurs sels d'addition avec les acides, pour leur utilisation comme facteurs de croissance des végétaux.

L'invention a aussi pour objet les compositions utilisées comme facteur de croissance des végétaux, caractérisées en ce qu'elles contiennent une quantité efficace de composés de formule (I) ou de leurs sels.

Les compositions selon l'invention peuvent se présenter sous forme de poudre, granulés, suspensions, émulsions, solutions contenant le principe actif, par exemple en mélange avec un véhicule et/ou un agent tensio-actif anionique, cationique ou non ionique assurant, entre autres, une dispersion uniforme des substances de la composition. Le véhicule utilisé peut être un liquide, tel que l'eau, l'alcool, les hydrocarbures ou autres solvants organiques, une huile minérale, animale ou végétale, ou une poudre, telle que le talc, les argiles, les silicates, le kieselguhr.

Les compositions solides, présentées sous forme de poudre pour poudrage, de poudres mouillables ou de granulés, peuvent être préparées par mélange du composé actif avec un solide inerte ou par imprégnation du support solide avec une solution du principe actif dans un solvant que l'on évapore ensuite.

Outre un véhicule et/ou agent tensio-actif, ces compositions peuvent contenir, comme principe actif, une ou plusieurs autres substances présentant des propriétés influant sur la croissance des plantes.

Les compositions de l'invention sont, bien entendu, utilisées à des doses suffisantes pour exercer leur activité de facteur de croissance des végétaux. Les doses de matière active dans les compositions varient notamment en fonction des végétaux à traiter, de la nature du sol, des conditions atmosphériques et de l'état d'avancement de la végétation.

Les compositions utilisées comme facteur de croissance, selon l'invention, contiennent en générale de 10 à 80% en poids et de préférence de 10 à 50% en poids de produits de formule (I).

Lors de l'utilisation des produits de formule (I) comme facteur de croissance des végétaux, les quantités de substance active appliquées varient entre 20 g/ha et 500 g/ha, et de préférence entre 40 et 120 g/ha.

L'invention a plus particulièrement pour objet les compositions destinées à l'usage agricole, pour être utilisées comme facteurs de croissance caractérisées en ce qu'elles renferment comme principe actif au moins l'un des composés suivants:

— l'O(2'méthyl 4-nitro phényl) hydroxylamine,
— l'O(4-nitro 3-méthoxy phényl) hydoxylamine,

ou encore l'un des composés suivants:

— l'O-(5-nitro 8-quinoléyl) hydroxylamine,
— l'O-(2-chloro 4-nitrophényl) hydroxylamine,
— l'O-(2-nitro 4-chlorophényl) hydroxylamine,
— l'O-(2,4-dichlorophényl) hydroxylamine,
— l'O-(4-nitro, 2-trifluorométhyl phényl) hydroxylamine,
— l'O-/2-(1RS hydroxyéthyl) 4-nitrophényl/ hydroxylamine;
— l'O-(3,4-dichlorophényl) hydroxylamine,
— l'O-(4-cyano phényl) hydroxylamine
— l'O-/3-(propyn-2-yloxy) 4-nitrophényl/ hydroxylamine,

ainsi que leurs sels d'addition avec les acides.

L'invention a également pour objet un procédé de préparation des composés de formule (I) et de leurs sels caractérisé en ce que l'on soumet un composé de formule:

Ar—Hal (II)

dans laquelle Ar conserve la même signification que précédemment et Hal représente un atome d'halogène, à l'action d'un composé de formule:

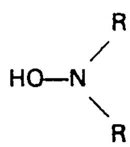

(III)

dans laquelle R représente un atome d'hydrogène ou les deux radicaux R forment un reste phtalimido, pour obtenir un composé de formule:

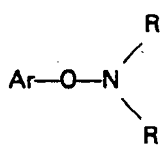

(IV)

dans laquelle Ar et R ont la signification précitée, correspondant à un composé de formule (I), dans le cas où R représente un atome d'hydrogène, composé de formule (IV) que l'on fait réagir, lorsque R est différent d'hydrogène, avec l'hydrazine, pour obtenir un composés de formule (I), puis fait réagir, si désiré, les composés de formule (I) obtenus avec un acide, pour en former les sels.

Dans un mode de réalisation préféré du procédé de l'invention:

— dans le cas où, dans la formule (III), R représente un atome d'hydrogène, on utilise un produit de formule (II) dans laquelle Hal représente un atome de fluor ou de brome;

— on fait réagir, le cas échéant, un sel d'hydroxylamine, par exemple le chlorhydrate, sur le composé Ar Hal en solution dans un alcool ou un milieu hydroalcoolique et en présence d'une base forte comme la soude, la potasse ou un alcoolate;

— on fait réagir, le cas échéant, le composé de formule (III) dans laquelle les radicaux R forment un reste phtalimido, avec une base forte comme l'hydrure de sodium ou de potassium, puis avec le composé de formule (II), ou encore directement avec le composé de formule (II) au sein d'un solvant inerte, comme par exemple, l'acétonitrile en présence d'une base tertiaire comme la triéthylamine ou la pyridine.

La formation des sels se fait selon les procédés classiques par action des acides sur les composés de formule (I) dans les solvants organiques usuels, par exemple les alcools ou les éthers ou les mélanges de ces solvants.

L'invention a également pour objet une variante du procédé pour préparer les produits de formule (I), qui peut être schématisé de la façon suivante:

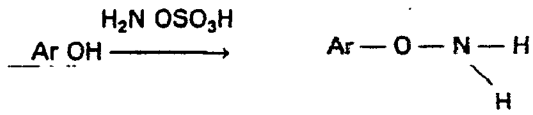


et qui est donc caractérisé en ce que l'on soumet un composé de formule:

$$Ar—OH \quad (V)$$

dans laquelle Ar garde sa signification précédente, à l'action d'un composé de formule:

$$H_2NOSO_3H \quad (VI)$$

pour obtenir le composé de formule (I) correspondant, que l'on fait réagir, si désiré, avec un acide pour en former le sel.

Les exemples suivants illustrent l'invention sans toutefois la limiter.

Exemple 1: O(2-méthyl 4-nitro phényl) hydroxylamine.

On introduit 8,35 g de chlorhydrate d'hydroxylamine, 84 $cm_3$ d'eau et 15, 5 g de 2-fluoro 5-nitro toluène dans 84 $cm_3$ d'alcool éthylene. On introduit ensuite en 14 minutes à o°C, 22 $cm_3$ de soude à 400 g/litre. On laisse remonter la température à 20°C et agite pendant 17 heures. On verse le mélange réactionnel dans l'eau, extrait au chlorure de méthylène, lave à l'eau, sèche la phase organique et amène à sec. On isole 1,1 g du produit recherché après chromatographie sur silice en éluant avec du chlorure de méthylène (F=118°C).

Préparation 1

Le 2-fluoro 5-nitro toluène utilisé comme produit de départ de l'exemple 1 est préparé de la façon suivante:

On refroidit à −15°C 30 g de 2-fluoro toluène et ajoute en 2 heures environ 33 $cm_3$ d'acide nitrique. On agite 1 heure à −15°C et laisse remonter la température à 20°C. On verse la solution réactionnelle sur de la glace. On extrait à l'éther, lave à l'eau, sèche la phase organique et amène à sec. Après rectification, on obtient 34,4 g du produit recherché (Eb: 100—101°C sous 10 à 11 mm de mercure).

Exemple 2: O(4-nitro 3-méthoxy phényl) hydroxylamine.

On refroidit à 0°C 10 g de 2-nitro 5-fluoro anisole, 38 $cm_3$ d'éthanol, 38 $cm_3$ d'eau, 4,88 g de chlorhydrate d'hydroxylamine. On introduit ensuite toujours à cette température en 15 minutes 13 $cm_3$ d'une solution de soude 10N. Une fois l'introduction de soude terminée, on laisse la température revenir à 20~25°C. On maintient sous agitation pendant 6 heures à la température ambiante, on essore le précipté formé et le sèche. On recristallise le produit obtenu dans le méthanol, on essore, sèche et isole 5,85 g du produit recherché fondant à 106~107°C.

Préparation 2

Le 2-nitro 5-fluoro anisole utilisé comme produit de départ est préparé de la façon suivante:

On dissout 12,6 g de 3-fluoro anisole dans 100 $cm_3$ d'anhydride acétique. On refroidit à −5°C~0°C et tout en agitant, on introduit goutte à goutte un mélange renfermant 32 $cm_3$ d'acide nitrique et 95 $cm_3$ d'anhydride acétique. On maintient le mélange réactionnel sous agitation pendant 1 heure à 0—5°C. On hydrolyse le mélange réactionnel en le versant sur de la glace. On essore, filtre et sèche le précipté obtenu. On obtient ainsi 6,77 g du produit recherché (F<50°C).

Exemple 3: O2-chloro 4-nitro phényl) hydroxylamine.

On introduit en 5 minutes à 5°C, 8,5 g de chlorhydrate d'hydroxylamine en solution dans 85 $cm_3$ d'eau, dans une solution renfermant 17,5 g de 3-chloro 4-fluoro nitro benzène et 170 $cm_3$ d'éthanol. On ajoute ensuite 22 $cm_3$ d'une solution de soude 10N. On agite à 20°C pendant 8 heures. On ajoute 100 $cm_3$ d'eau et essore la suspension obtenue. On lave à l'eau et sèche sous pression réduite. On obtient 9 g de produit que l'on purifie par cristallisation dans le méthanol. On obtient ainsi 7,6 g de produit recherché fondant à 125~126°C.

Préparation 3

Le 3-chloro 4-fluoro nitro benzène utilisé comme produit de départ est préparé comme suit:

On melange 120 $cm_3$ de diméthylsulfoxyde, 80 $cm_3$ de benzène et 35 g de fluorure de potassium. On

ajoute à ce moment, 50 g de 3,4-dichloronitro benzène. On agite, chauffe à 180—185°C pendant 2 heures. On entraîne à la vapeur d'eau et extrait le distillat avec de l'éther isopropylique. On réunit les phases organiques, les sèche et les distille sous pression de 3—4 mm de mercure à 40°C. On rectifie le résidue obtenu sous pression de 15 mm de mercure. On obtient 29 g du produit recherché fondant à 41~43°C.

Exemple 4: O(2-nitro 4-chloro phényl) hydroxylamine.

On agite à 5°C une solution renfermant 8,7 g de 2-fluoro 5-chloro nitro benzène dans 85 $cm_3$ d'éthanol. On ajoute 4,4 g de chlorhydrate d'hydroxylamine dans 45 $cm_3$ d'eau. On ajoute 11 $cm_3$ d'une solution de soude 10N. On agite ensuite 6 heures à 20°C. On ajoute 45 $cm_3$ d'eau, essore le précipité, le lave à l'eau. On extrait le précipité par du chlorure de méthylène, réunit les phases organiques, lave à l'eau, sèche et distille à 40°C sous pression réduite (3—4 mm Hg). On obtient 2,1 g de produit recherché fondant à 125~126°C.

Préparation 4

Le 2-fluoro 5-chloro nitro benzène utilisé comme produit de départ de l'exemple est préparé comme suit:

On chauffe en agitant à 140°C, un mélange renfermant 120 $cm_3$ de diméthylsulfoxyde, 80 $cm_3$ de benzène, 35 g de fluorure de potassium et 50 g de 2,5-dichloro nitro benzène. On porte à 180~185°C pendant 3 heures. On refroidit et entraîne à la vapeur d'eau. On extrait le distillat à l'éther isopropylique. On réunit les extraits, les sèche et les distille à 40°C sous pression réduite. Après rectification sous 14 mm de mercure, on obtient 10,1 g de produit recherché ($n_D^{20}$ = 1,5558).

Exemple 5: O(5-nitro 8-quinoléyl) hydroxylamine.

On chauffe à 40°C en agitant 9,6 g de 8-fluoro 5-nitro quinoléine, 300 $cm_3$ de méthanol et 4,4 g de chlorhydrate d'hydroxylamine. On refroidit la solution obtenue à 5°C et introduit en 30 minutes à 5—10°C, 11 $cm_3$ d'une solution de soude 10N. On agite 6 heures à 20°C. On essore, extrait le précipité au chlorure de méthylène, sèche et distille sous pression réduite. On ajoute 15 $cm_3$ de chlorure de méthyléne et obtient 1,6 g de produit recherché fondant à 228~230°C.

Préparation 5

La 8-fluoro 5-nitro quinolèine utilisée comme produit de départ est préparée de la façon suivante:

On refroidit à −5°C 30 $cm_3$ d'acide sulfurique concentré, on agite et introduit 15 $cm_3$ d'acide nitrique. On introduit ensuite entre −5 et 0°C, en 30 minutes et sous agitation, 16 g de 8-fluoro quinolèine. On agite ensuite pendant 3 heures le mélange réactionnel à 0°C puis on laisse revenir à la température ambiante. On agite pendant 2 heures à cette température. On verse dans un mélange d'eau et de glace, essore le précipité obtenu, le met en suspension dans l'eau et alcanilise par une solution de carbonate de soude à 10%. On extrait au chlorure de méthylène, sèche et distille à sec. On obtient 12 g du produit recherché (F=132—133°C).

En opérant comme précédemment — à partir des produits de départ II, on a obtenue les produits finals (I):

Produits II — Produits I

Exemple 6: O(2-nitro 4-carboxyphényl) hydroxylamine.

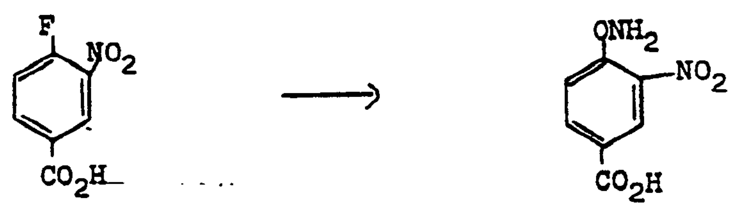

PF=215°C

Exemple 7: O-benzothiazolyl hydroxylamine.

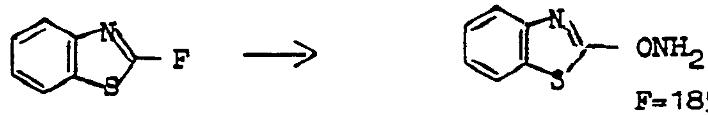

F=185~186°C

Produits II Produits I

Exemple 8: O-(4-nitro 2-bromophényl) hydroxylamine.

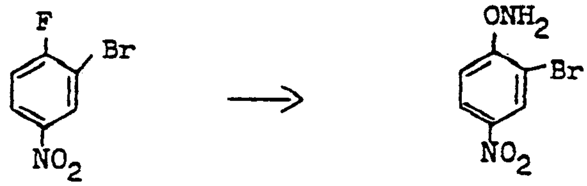

F=122°C

Exemple 9: O-(4-nitro 3-méthylphényl) hydroxylamine.

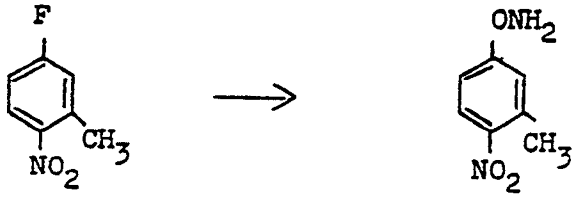

F=100°C

Exemple 10: O-(4-nitro 3-bromophényl) hydroxylamine.

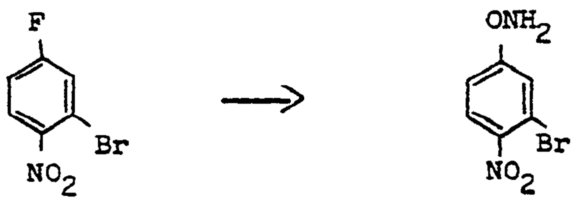

F=95°C

Exemple 11: O-(4-nitro 2-fluorophényl) hydroxylamine.

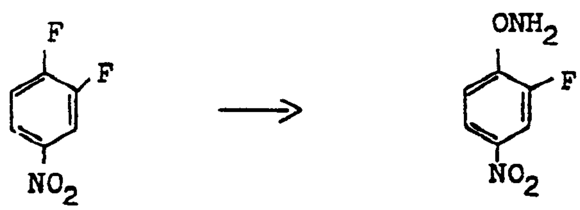

F=125°C

Exemple 12: O-(4-nitro 3-éthoxycarbonylphényl) hydroxylamine.

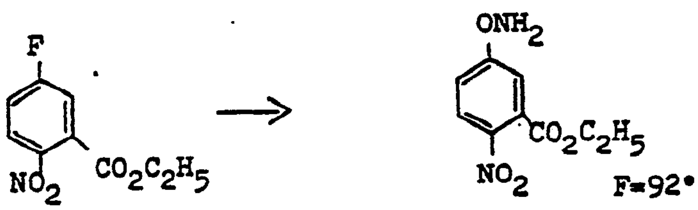

F=92°C

Exemple 13: O-(2,4-dinitro 5-méthylphényl) hydroxylamine.

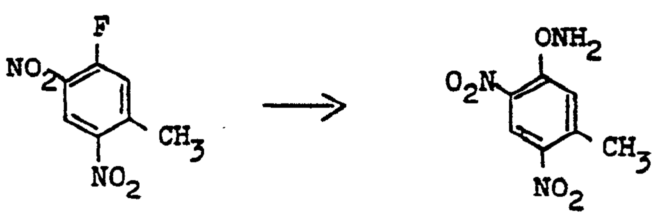

F=105°C

Produits II Produits I

Exemple 14: O-/4-nitro 2-(1-RS-hydroxyéthyl) phényl/ hydroxylamine.

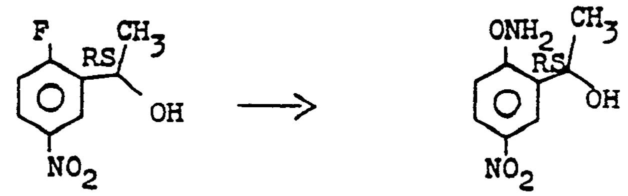

F=120°C

Exemple 15: O-(4-nitro 2-éthoxycarbonylphényl) hydroxylamine.

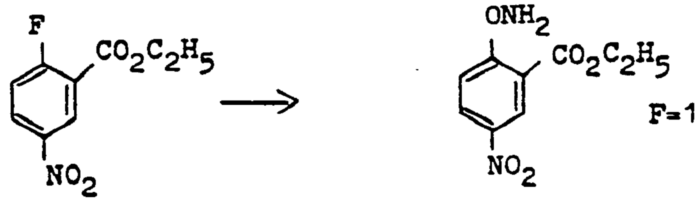

F=116~117°C

Exemple 16: O-(4-nitro 2-diéthylcarbamoylphényl) hydroxylamine.

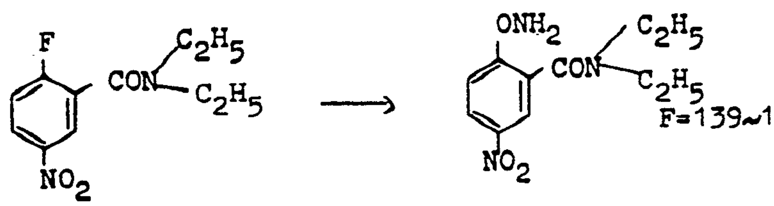

F=139~140°C

Exemple 17: O-(4-nitro napht-l-yl) hydroxylamine.

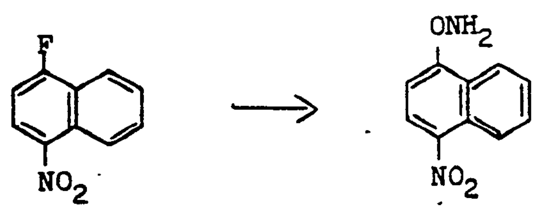

F=108-110°C

Exemple 18: O-(4-nitro 2-mèthoxycarbonylphényl) hydroxylamine.

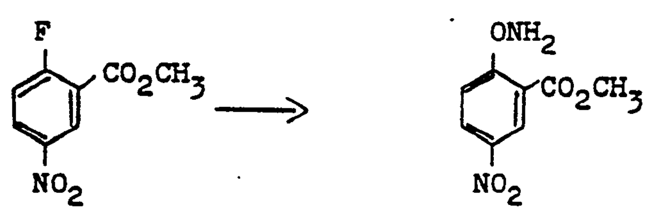

F=175~180°C

Exemple 19: O-(nitro 3-acétylaminophényl) hydroxylamine.

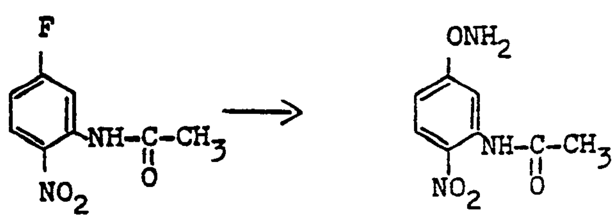

F=150-152°C

Produits II | Produits I

Exemple 20: O-(4-nitro 3-aminophényl) hydroxylamine.

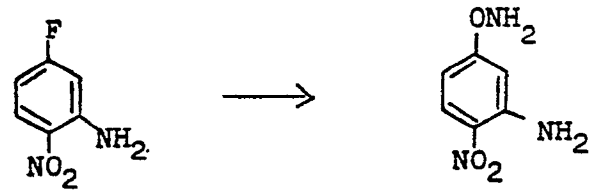

F=190~191°C

Exemple 21: O-(4-éthoxycarbonyl 2-nitrophényl) hydroxylamine.

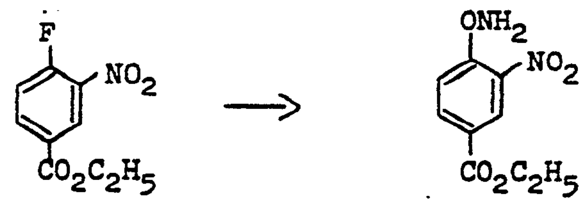

F=102°C

Exemple 22: O-(4-nitro 3-n-pentyloxyphényl) hydroxylamine.

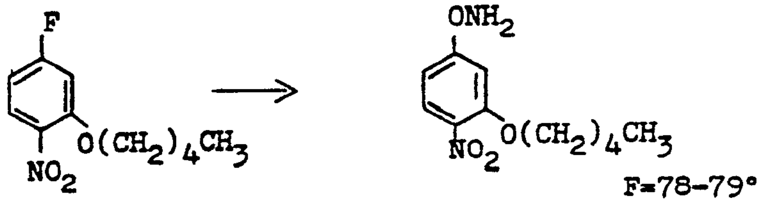

F=78-79°C

Exemple 23: O-(4-nitro 3-allyloxyphényl) hydroxylamine.

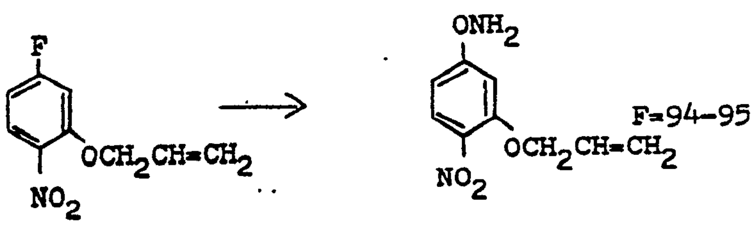

F=94-95°C

Exemple 24: O-(4-nitro 3-isopropyloxyphényl) hydroxylamine.

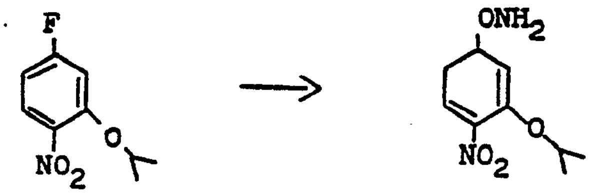

F=104°C

Exemple 25: O-(4-nitro 3-éthoxyphényl) hydroxylamine.

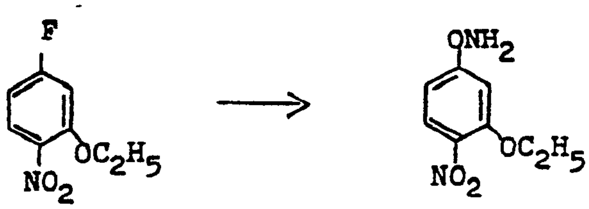

F=97~98°C

Produits II | Produits I

Exemple 26: O-(4-nitro 3-n-butyloxyphényl) hydroxylamine.

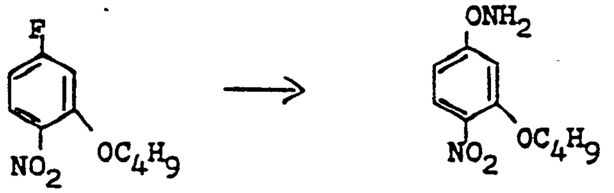

F=75-76°C

Exemple 27: O-(4-acétyl 2-nitrophényl) hydroxylamine.

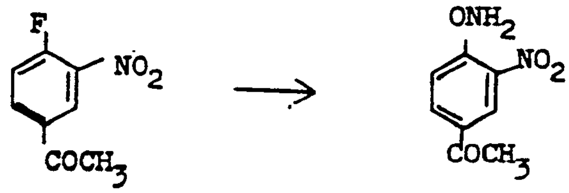

F=132°C

Exemple 28: O-/4(nitro 3-(1-RS-hydroxyéthyl) phényl/ hydroxylamine.

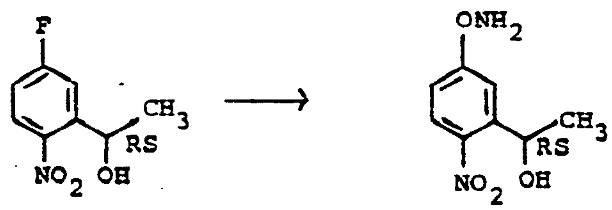

F=118-120°C

Exemple 29: O-/4-nitro 3-(3,3-dichloroallyloxy) phényl/ hydroxylamine.

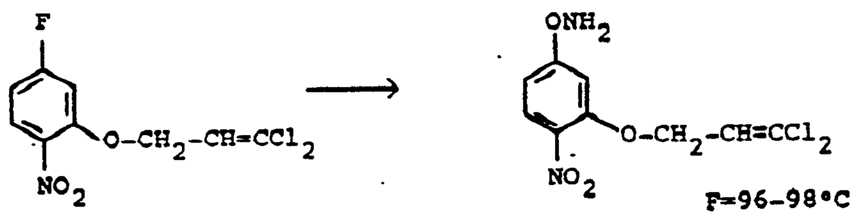

F=96-98°C

Exemple 30: O-(4-nitro 3-méthylaminophényl) hydroxylamine.

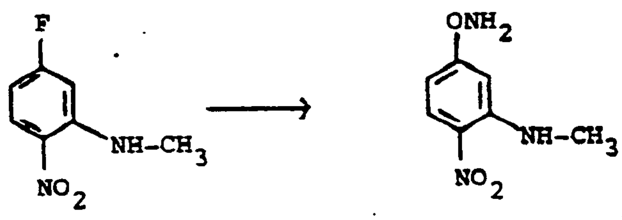

F=157-158°C

Exemple 31: O-(4-nitro 3-diméthylaminophényl) hydroxylamine.

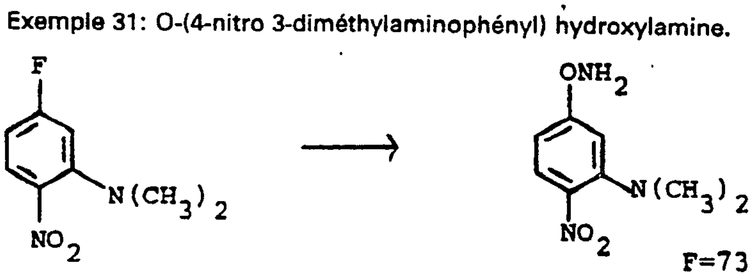

F=73°C

Produits II Produits I

Exemple 32: O-/4-nitro 3-(4-nitrophényloxy) phényl/ hydroxylamine.

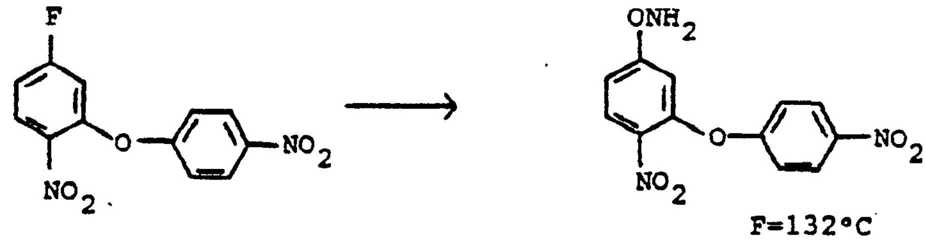

F=132°C

Les préparations des produits de départ des examples 6 à 32 sont indiquées ci-après.

Préparation 6

Acide 3-nitro 4-fluoro benzoïque.

On refroidit à 0°C, 300 $cm_3$ d'acide sulfurique concentré et ajoute 150 $cm_3$ d'acide nitrique (d=1,42). On maintient le mélange réactionnel à 0°C et ajoute en 30 mn 50 g d'acide p-fluoro benzoïque. On agite pendant 1 heure à 0°C et laisse remonter la température à 20°C puis agite pendant 16 heures à cette température. On verse la solution réactionnelle sur de la glace, on essore, lave et sèche le précipité obtenu. On obtient 47,6 g du produit recherché (F=123—124°C).

Préparation 7

3-bromo 4-fluoro nitro benzène.

On mélange à 20°C—28,2 g de parafluoronitro benzène et 10,4 cc de brome. On refroidit à 0°C et ajoute lentement 20 cc d'eau et 180 cc d'acide sulfurique concentré. On ajoute ensuite 34 g de sulfate d'argent. On laisse remonter la température à 20—25°C et agite 16 heures. On coule le mélange réactionnel dans l'eau, essore l'insoluble, le lave à l'eau et au chlorure de méthylène. On décante, extrait le filtrat, le lave à l'eau et le sèche sur sulfate de magnésium, on filtre le sulfate et amène à sec le filtrat. On dissout le produit dans l'éther isopropylique, le recristallise. On essore et lave le produit recherché. On obtient ainsi 8,2 g du produit recherché fondant à 59°C.

Les produits de départ des exemples 9 à 12 ont été préparés selon les schémas suivants en utilisant le procédé de nitration déjà décrit.

Préparation 8

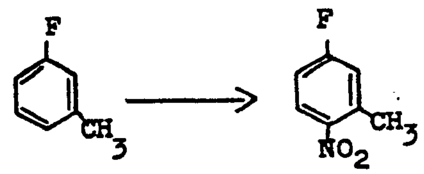

(Ex. 9)

Préparation 9

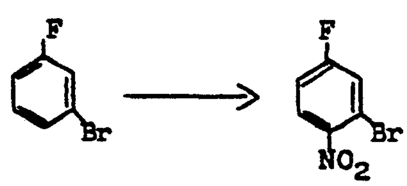

(Ex. 10)

F=42°C

Préparation 10

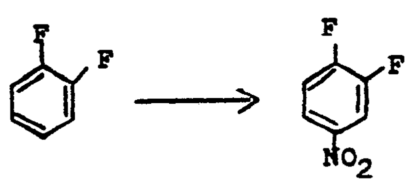

(Ex. 11)

Eb=80°C/10mm Hg

Préparation 11

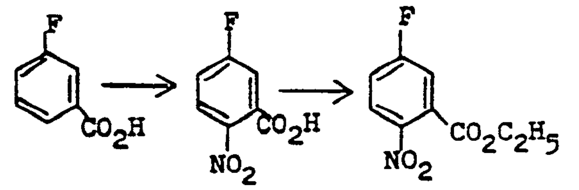

(Ex. 12)

F=139°C F<50°C

Préparation 12

3-fluoro 4,6-dinitro toluène

On refroidit à −15°C une solution renfermant 30 g de métafluoro toluéne et 60 $cm_3$ d'acide sulfurique 36°B. On ajoute en 2 heures, sans dépasser 0°C, 30 $cm_3$ d'acide nitrique d=1,49. On agite 1 heure à 0°C, verse dans de la glace, extrait au chlorure de méthyléne. On lave à l'eau, sèche et amène à sec. On empâte le résidu obtenu dans l'éther isopropylique, essore et sèche. On obtient 21,8 g de produit fondant à 76°C.

Préparation 13

1(2-fluoro 5-nitro phényl) éthanol

Stade A: 2-fluoro 5-nitro acétophénone

On introduit à 0~10°C 23,7 g d'orthofluoroacétophénone dans 113 cc d'acide nitrique (d=1,52). On laisse en contact 30 minutes à −10°C~0°C. On hydrolyse le mélange réactionnel dans de l'eau et de la glace. On essore le précipité formé, le lave à l'eau et le sèche. On obtient 26,98 g de produit recherché (F=56~58°C).

Stade B: 1(2-fluoro 5-nitro phényl) éthanol

On introduit 1,04 g d'hydroborure de sodium dans une solution à 0°, + 5° renfermant 10 g de 2-fluoro 5-nitro acétophénone et 100 $cm_3$ de méthanol. On maintient 1 heure en contact a 0°, + 5° et verse le mélange réactionnel sur de la glace, on extrait au dichloro méthane, sèche, filtre et amène à sec sous pression réduite. On obtient ainsi 9,47 g de produit recherché fondant à une température inférieure à 50°C.

Préparation 14

2-fluoro 5-nitro benzoate d'éthyle

Stade A: 2-fluoro benzoate d'éthyle

On introduit 35 g de chlorure de 2-fluoro benzoyle dans 400 $cm_3$ d'éthanol. On agite pendant trois heures la solution obtenue. On laisse au repos pendant une nuit. On distille à 50°C sous pression de 3—4 centimètres de mercure. On reprend dans le chlorure de méthylène, on lave avec une solution aqueuse de carbonate acide de sodium à 5% et à l'eau. On sèche et distille le produit obtenu. On rectifie sous une pression de 14 mm de mercure. On obtient 34 g de produit recherché ($Eb_{14mm}$ = 90—91°C).

Stade B: 2-fluoro 5-nitro benzoate d'éthyle

On introduit en refroidissant 31,8 g de 2-fluoro benzoate d'éthyle dans 60 $cm_3$ d'acide sulfurique concentré. On agite, refroidit à 0°C et introduit en 30 minutes entre 0 et 5°C, 15,9 g d'acide nitrique (d=1,5). On agite deux heures à +5, 10°C. On verse sur un mélange de glace et d'eau. On extrait au chlorure de méthylène. On réunit les phases organiques, les lave avec une solution de carbonate acide de sodium à 5% et à l'eau. On sèche et distille. On obtient un produit que l'on triture dans l'hexane, essore et sèche sous pression réduite. On obtient 37,7 g de produit recherché fondant à 53~54°C.

Préparation 15

N,N-diéthyl 2-fluoro 5-nitro benzamide

Stade A: chlorure de 2-fluoro 5-nitro benzoyle

On chauffe au reflux pendant 2 heures une solution renfermant 42 g d'acide 2-fluoro 5-nitro benzoïque préparé selon le procédé décrit dans Chim. et Ind. *1970* 892 (F=139—140°C) et 120 $cm_3$ de chlorure de thionyle. On distille à 40°C sous pression réduite (3—4 cm/Hg), reprend dans le benzène et distille à nouveau. On obtient 44 g de chlorure de 2-fluoro 5-nitro benzoyle fondant à 59~60°C.

Stade B: N,N-diéthyl 2-fluoro 5-nitro benzamide

On agite à 5°C 32 g de diéthylamine et 350 $cm_3$ de benzène anhydre. On ajoute en 30 minutes à 5—10°C, 40,7 g de chlorure de 2-fluoro 5-nitro benzoyle et 100 $cm_3$ de benzène. On agite 1 heure à 20°C. On filtre, lave le benzène. On lavel le filtrat à l'eau, à l'acide chlorhydrique N, à l'eau, avec une solution aqueuse de bicarbonate de soude à 10%, à l'eau et sèche. On traite au charbon actif, on filtre et distille sous pression réduite à 50°C. On obtient 38 g d'un produit que l'on chromatographie sur silice en éluant par le mélange chlorure de méthylène-acétate d'éthyle (9—1). On obtient ainsi 33 g de produit recherché ($n_D^{34}$=1,5330).

Préparation 16

2-fluoro 5-nitro benzoate de méthyle

On chauffe au reflux pendant 1 heure 20 g d'acide 2-fluoro 5-nitro benzoïque en solution dans 60 $cm_3$ de chlorure de thionyle. On distille sous pression réduite. On reprend dans 50 $cm_3$ de benzène, distille sous pression réduite. On répète plusieurs fois cette opération. On obtient le chlorure d'acide que l'on verse dans 400 $cm_3$ de méthanol. On agite la solution obtenue pendant 2 heures à 25~30°C. On laisse au repos pendant une nuit. On distille sous pression réduite à 50°C. On reprend dans l'éther isopropylique, lave, sèche et distille sous pression réduite. On obtient 13 g de produit recherché (F=52~53°C).

Les produits de départ des exemples 17 et 19 ont été préparés selon le schéma suivant:

Préparation 17

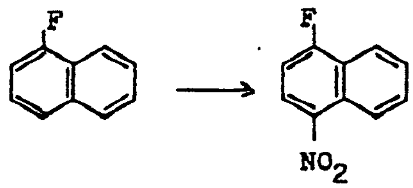

F=75~76°C

Préparation 18

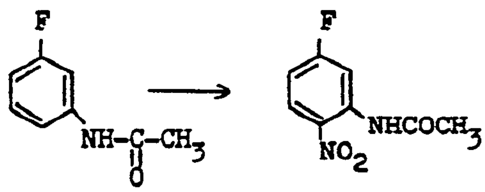

F=40°C

Préparation 19

3-fluoro 6-nitro aniline

On chauffe pendant 30 minutes environ à 110~120°C, 20 g de 3-fluoro 6-nitro acétanilide, dans 200 $cm_3$ d'une solution d'acide chlorhydrique 6N. On verse la solution réactionnelle dans un mélange d'eau et de glace. On essore le précipité formé et le purifie par recristallisation dans l'éther isopropylique. On obtient 7,5 g de produit recherché fondant à 99°C.

Preparation 20

3-nitro 4-fluoro phényl carboxylate d'éthyle

On dissout 10 g — d'acide 3-nitro 4-fluoro benzoïque (préparation 6) dans 50 $cm_3$ d'éthanol. On ajoute 2 $cm_3$ d'acide sulfurique concentré. On porte au reflux pendant 3 heures. On ajoute 8 $cm_3$ d'acide sulfurique concentré et continue le reflux pendant 16 heures. On refroidit à 20°C et verse dans l'eau sous agitation. On essore, lave et sèche le précipité obtenu. On obtient ainsi 10,5 g de produit recherché fondant à une température de 50°C.

Les produits de départ des exemples 22 à 27 ont été préparés de la façon suivante:

Préparation 21

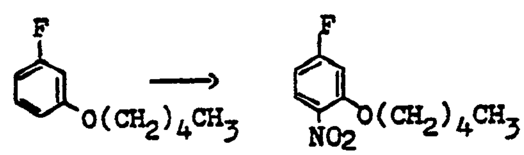

Le 3-fluoro n-pentyloxy benzène utilisé comme produit de départ a été préparé par éthérification du 3-fluoro phénol ($n_D^{33}$=1,4741).

Préparation 22

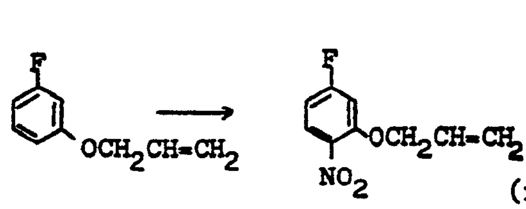

($n_D^{23}$=1,5432)

Le 3-fluoro allyloxy benzène a été préparé par action du bromure d'allyle sur le 3-fluoro phénol ($n_D^{23}$=1,4985).

Préparation 23

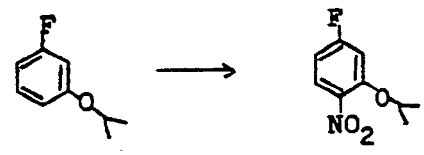

Le 3-fluoro isopropyloxy benzène a été préparé par action de l'iodure d'isopropyle sur le 3-fluoro phénol.

Préparation 24

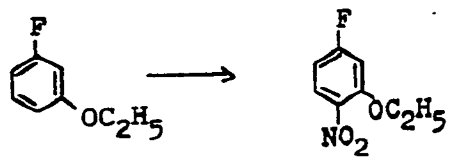

F=28-30°C

Le 3-fluoro éthoxy benzéne a été préparé par action de l'iodure d'ethyle sur le 3-fluoro phénol ($n_D^{28,5}$=1,4823).

Préparation 25

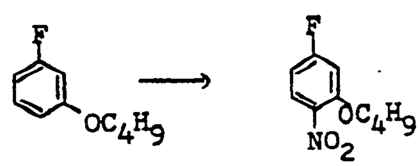

($n_D^{26}$=1,5180)

Le 3-fluoro n-butoxy benzène de départ a été préparé par action de l'iodure de n-butyle sur le 3-fluoro phénol ($n_D^{30}$=1,4762).

Préparation 26

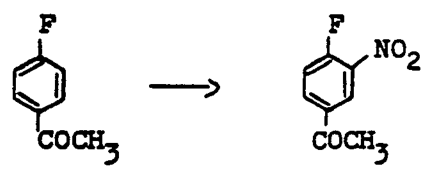

F= 56-58°

Préparation 27
Le 4-nitro 3-(1-RS-hydroxyéthyl) fluorobenzène a été préparé par nitration de la m-fluoro acétophénone puis réduction par l'hydroborure de sodium dans des conditions classiques.

Préparation 28
Le 4-nitro 3-(3,3-dichloroallyloxy) fluorobenzène a été préparé par action du chlorure de 3,3-dichloroallyle sur le phénol correspondant, en présence d'une base.

Préparation 29
Les 4-nitro 3-méthyl et 3,3-diméthylamino fluorobenzènes ont été préparés par nitration du 2-amino fluorobenzène protégé par un radical acétyl, déprotection du radical amino et enfin méthylation dudit radical amino libre.

Préparation 30
Le 4-nitro 3-(4-nitrophényloxy) fluorobenzène a été préparé par action du para-chloronitrobenzène sur le méta-fluorophénol en présence d'hydrure de sodium dans le tétrahydrofuranne, puis par nitration du composé ainsi obtenu.

Exemple 33: O-(4-nitro 2-trifluorométhylphényl) hydroxylamine.

Stade A: N(4-nitro 2-trifluorométhyl phénoxy) phtalimide

On mélange à 20°C, 11,5 g d'hydrure de sodium à 50% dans l'huile et 300 $cm_3$ de diméthyl formamide anhydre. On refroidit la suspension à + 5, +10°C et introduit 36,5 g de N-hydroxy phtalimide. On agite 30 minutes à 20°C et ajoute en 10 minutes 50 g de 4-nitro 2-trifluorométhyl chloro benzène. On chauffe à 80~85°C la suspension obtenue pendant 1 heure. On refroidit à 20°C et verse dans de la glace. On agite pendant 1 heure. On essore la suspension et lave à l'eau. On sèche et obtient 60,7 g du produit recherché fondant à 190°C.

Stade B: O(4-nitro 2-trifluorométhyl phényl) hydroxylamine

On dissout 5 g de produit préparé au stade A dans 50 $cm_3$ d'éthanol. On ajoute ensuite 2 $cm_3$ d'hydrazine hydratée. On agite 1 heure 30 à 20°C et on verse la suspension dans de la glace; on acidifie à pH1 avec une solution d'acide chlorhydrique. On essore, lave au chlorure de méthylène. On décante le filtrat, extrait au chlorure de méthylène, lave les phases organiques réunies à l'eau, les sèche et amène à sec. On chromatographie sur silice le résidu obtenu en éluent avec du chlorure de méthylène. On empâte à chaud dans l'hexane et le produit obtenu puis refroidit. On essore, lave avec de l'hexane et sèche à 40°C sous pression réduite. On obtient 2,1 g de produit recherché fondant à 106°C.

En opérant comme précédemment, on a obtenu les produits (I) à partir des produits II et III, en passant intermédiairement par les produits IV.

Exemple 34: O-(8-nitro 5-quinoléyl) hydroxylamine.

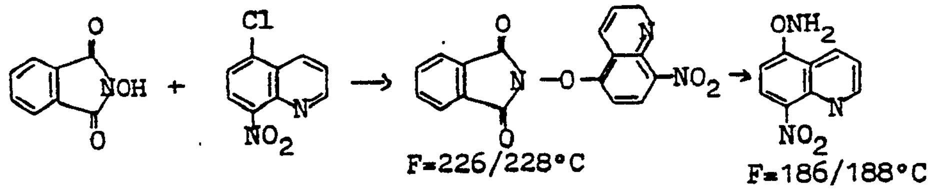


Exemple 35: O-(4-nitro 3-trifluorométhylphényl) hydroxylamine.

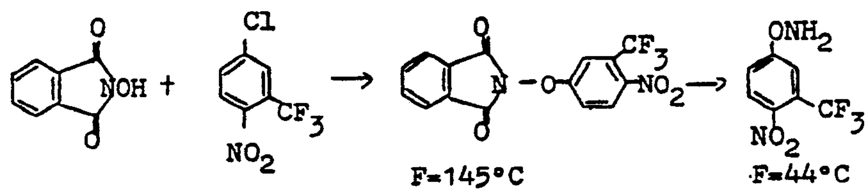


Exemple 36: O-(7-chloro 4-quinoléyl) hydroxylamine.

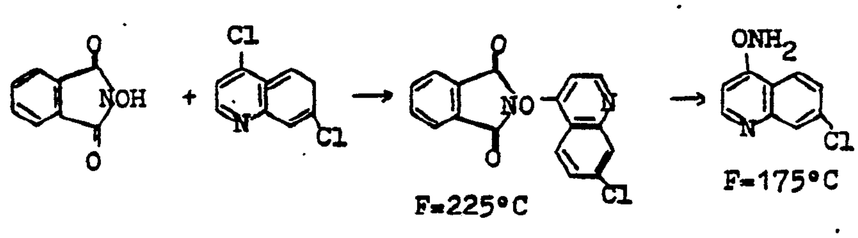


Exemple 37: O-(5-nitro 6-quinoléyl) hydroxylamine.

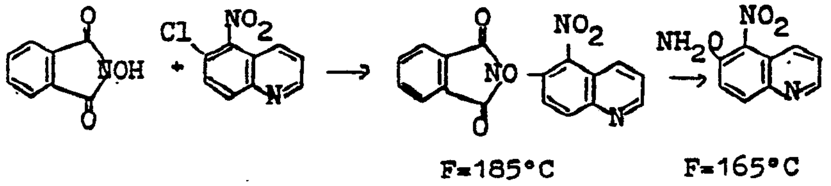

Exemple 38: Chlorhydrate de O-(4-nitrophénylméthyl) hydroxylamine.

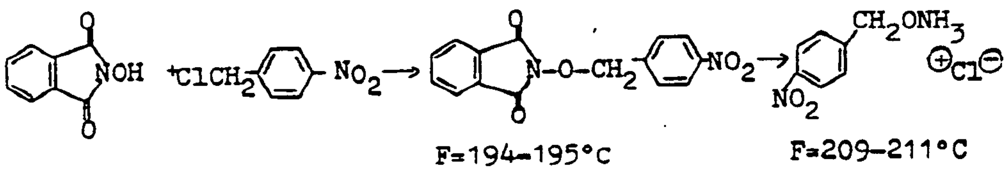

F=194–195°C F=209–211°C

Exemple 39: O-(2-nitrophényl) hydroxylamine.

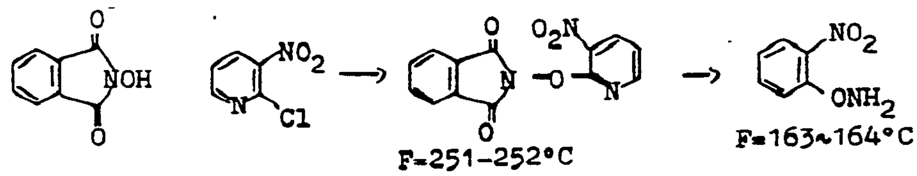

F=251–252°C F=163~164°C

Exemple 40: O-(4-cyano 2-nitrophényl) hydroxylamine.

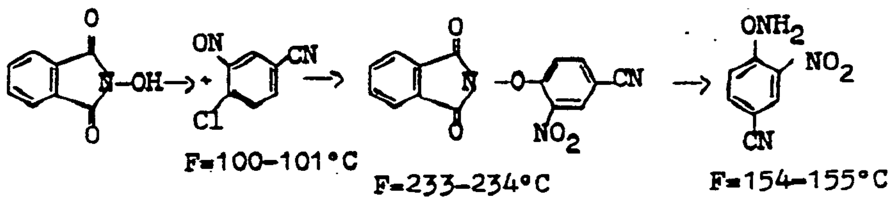

F=100–101°C F=233–234°C F=154–155°C

Exemple 41: O-(4-méthylsulfonyl 2-nitrophényl) hydroxylamine.

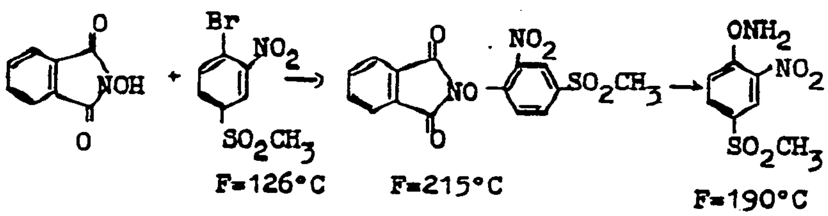

F=126°C F=215°C F=190°C

Exemple 42: O-(4-nitro 2-isopropylphényl) hydroxylamine.

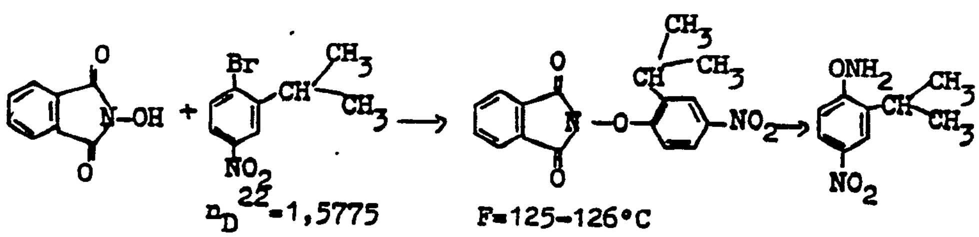

$n_D^{22}$=1,5775 F=125–126°C F=81~82°C

Exemple 43: O-(4-fluoro 2-nitrophényl) hydroxylamine.

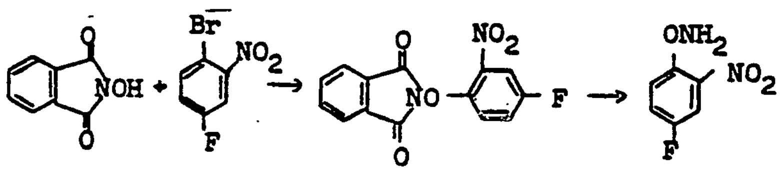

F=40–41°C F=181~182°C F=87~88°C

Exemple 44: O-(8-chloro 4-quinoléyl) hydroxylamine.

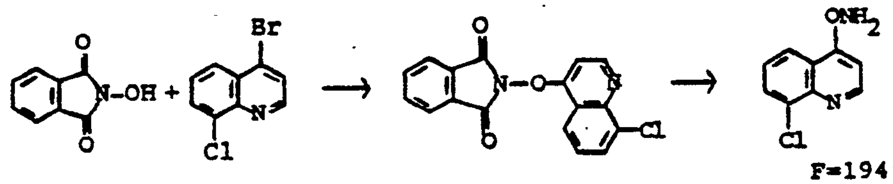

F=194°C

Exemple 45: O-(8-trifluorométhyl 4-quinoléyl) hydroxylamine.

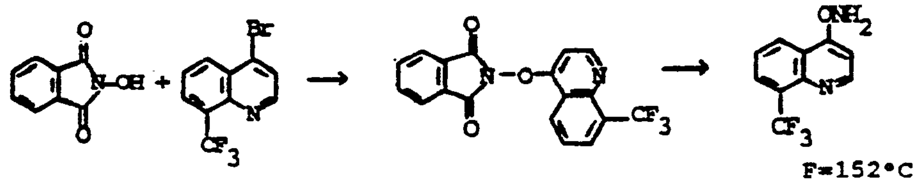

F=152°C

Exemple 46: O-(4-nitro 3-éthylphényl) hydroxylamine.

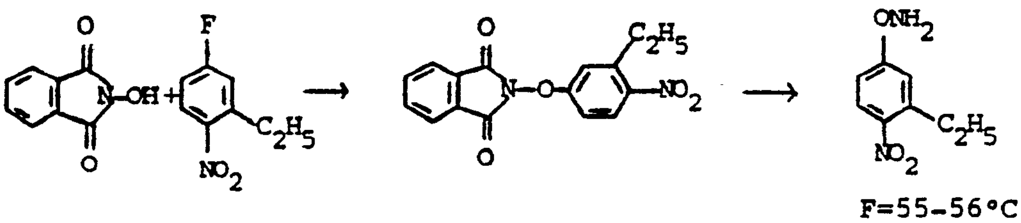

F=55-56°C

Exemple 47: O-/4-nitro 3-(phénylsulfonylméthyl) phényl/ hydroxylamine.

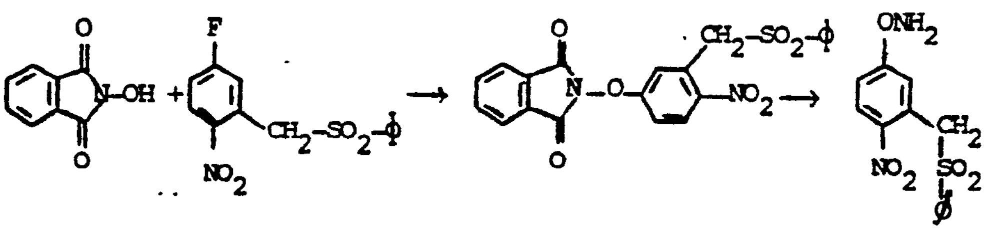

F=195-196°C

Préparation 31

5-chloro 8-nitro quinoléine

On porte au reflux 25,2 g de 3-chloro 6-nitro aniline, 21 g d'acide arsenique, 35,1 $cm_3$ de glycérine. On ajoute ensuite en maintenant la température à 20~30°C, 25,5 $cm_3$ d'acide sulfurique 66°B. On chauffe à 140°C pendant 8 heures. On verse sur de la glace. On essore le précipité formé et le dissout dans le chlorure de méthylène. On neutralise le milieu réactionnel et extrait les liqueurs mères au chlorure de méthylène. On réunit toutes les phases chlorométhylèniques, les sèche, les traite au charbon actif et les amène à sec. On obtient 10 g de produit que l'on purifie par chromatographie sur silice en éluant avec du chlorure de méthylène. On récupère ainsi 8,61 g de produit attendu fondant à 136~138°C.

Préparation 32

2-nitro 4-fluoro bromo benzène

Stade A: 2-nitro 4-fluoro acètanilide

On introduit 13,8 g de 4-fluoro acétanilide préparé selon le procédé décrit dans Acta Chemica Scand. *1976* page 141 dans 35 $cm_3$ d'acide sulfurique concentré. On refroidit à 0°C et ajoute, en 30 minutes, entre 0 et 5°C, 6,5 $cm_3$ d'acide nitrique (d=1,42). On agite 2 heures à 0° ± 5°C. On introduit ensuite à 0°C, 10 $cm_3$ d'annhydride acétique. On agite 1 heure à 0°C puis on verse sur un mélange eau-glace en agitant. On essore le précipité formé, le lave et le sèche. On obtient 14 g du produit recherché (F=70~71°C).

Stade B: 2-nitro 4-fluoro aniline

On verse 40 g de 2-nitro 4-fluoro acétanilide dans 100 $cm_3$ d'une solution d'acide chlorhydrique 8N. On agite et chauffe au reflux pendant 1 heure. Il y a cristallisation. On laisse refroidir à 20°C puis glace 1 heure. On essore, lave à l'eau jusqu'à neutralité et sèche sous pression réduite. On obtient 32,2 g de produit brut (F=94~95°C) que l'on purifie par mise en suspension dans 500 $cm_3$ de chlorure de méthylène et agitation avec 300 $cm_3$ d'une solution de soude N, décantation, lavage à l'eau, séchage et distillation sous pression réduite. On obtient 27,2 g de produit recherché fondant vers 94~95°C.

Stade C: 2-nitro 4-fluoro bromo benzène

On agite une suspension renfermant 21,6 g de 2-nitro 4-fluoro aniline, 69 $cm_3$ d'acide bromhydrique à 48% et 138 $cm_3$ d'eau. On refroidit à 0°C. On introduit 10 g de nitrite de sodium et 30 $cm_3$ d'eau. On agite 10 minutes à 0°C. On introduit la solution obtenue dans une solution à 60°C de 30 g de bromure cuivreux dans 105 $cm_3$ d'acide bromhydrique à 48% en agitant. On agite encore pendant 1 heure à 55~60°C. On dilue à l'eau glacée, extrait à l'éther isopropylique. On réunit les phases organiques, les sèche et les distille sous pression réduite. On chromatographie le résidu obtenu sur silice en éluant par le chlorure de méthylène. On distille le produit obtenu. On obtient un produit cristallisé que l'on sèche sous pression réduite. On obtient ainsi 20,2 g de produit recherché (F=40~41°C).

Préparation 33

3-nitro 4-chloro benzonitrile

Le produit a été préparé selon le procédé de nitration décrit précédemment à partir du 4-chloro benzonitrile (F=100~101°C).

Préparation 34

2-nitro 4-(méthyl sulfonyl) bromo benzène

Le produit a été préparé selon le procédé de nitration décrit précédemment à partir du 4-méthylsulfonyl bromo benzène (F=126°C).

Préparation 35

3-isopropyl 4-bromo nitro benzène

Stade A: 2-isopropyl acétanilide

On introduit 86 g d'anhydride acétique dans une solution renfermant 108 g de 2-isopropyl aniline et 250 $cm_3$ de benzène. On ajoute alors 86 g d'anhydride acétique. On agite pendant 16 heures à 20°C. On lave à l'eau avec une solution aqueuse de carbonate acide de sodium puis à l'eau. On sèche et distille sous pression réduite pour chasser le benzène. On triture le résidu dans l'hexane, glace et essore le produit obtenu. On lave à l'hexane et sèche sous pression réduite. On obtient 115 g de produit recherché (F=67—68°C).

Stade B: 2-isopropyl 4-nitro acétanilide

On refroidit à −10°C une solution renfermant 71 g de produit préparé au stade A, 300 $cm_3$ d'anhydride acétique, 100 $cm_3$ d'acide acétique. On introduit ensuite en 30 minutes entre −5 et −10°C, 32 $cm_3$ d'acide nitrique (d=1,50). On agite 30 mn à −5°C puis 3 heures à 0 + 2°C. On verse sur un mélange d'eau et de glace en agitant. On agite pendant 1 heure, essore le précipité obtenu, le lave à l'eau et le sèche. On obtient 70 g de produit que l'on chromatographie sur silice en éluant par le mélange chlorure de méthylène-acétate d'éthyle (7—3). On obtient ainsi 19,4 g de produit recherché (F=160—161°C).

Stade C: 2-isopropyl 4-nitro aniline

On chauffe au reflux pendant 2 heures une solution renfermant 19 g de 2-isopropyl 4-nitro acétanilide et 60 $cm_3$ d'acide chlorhydrique 8N. On refroidit et essore le précipité obtenu. On le met en suspension dans l'eau et alcalinise à l'aide d'une solution aqueuse de carbonate de sodium à 10%. On extrait à l'éther isopropylique. On dilue les liqueurs mères chlorhydriques à l'eau, alcalinise et extrait à l'éther isopropylique. On réunit les phases organiques, les lave à l'eau et les sèche. On distille sous pression réduite le produit obtenu. On obtient 11,7 g de produit recherché.

Stade D: 3-isopropyl 4-bromo nitro benzène

*Préparation du bromure de diazonium*

On refroidit à −5°C une suspension renfermant 18,5 g de 2-isopropyl 4-nitro aniline, 120 $cm_3$ d'une solution d'acide bromhydrique à 48% et 80 $cm_3$ d'eau. On introduit ensuite entre −5°C et 0°C en 20 minutes 7,5 g de nitrite de sodium et 20 $cm_3$ d'eau. On agite à 0°C pendant 15 minutes et conserve à 0°C.

On agite et chauffe à 50~55°C une solution de 22 g de bromure cuivreux dans 80 $cm^3$ d'acide bromhydrique à 48%. On introduit à 50~55°C la solution de bromure de diazonium préparée ci-dessus. On agite 1 heure à 55~60°C. On refroidit, ajoute de l'eau et extrait au chlorure de méthylène. On réunit les phases organiques, les lave avec une solution de soude N, avec de l'eau et les sèche. On distille sous pression réduite à 50°C. On rectifie sous pression réduite et obtient 20,4 g de produit recherché ($Eb_{8mm}$ = 150—152°C).

Préparation 36

Le 4-nitro 3-éthyl fluorobenzène a été préparé par nitration du 3-éthyl fluorobenzène.

Préparation 37

Le 4-nitro 3-(phénylsulfonylméthyl) fluorobenzène a été préparé par action du chlorure de phénylsulfonylméthyle sur le 4-nitro fluorobenzène dans le diméthylsulfoxyde en présence de terbutylate de potassium.

Les 4-bromo 8-chloro et 8-trifluorométhyl quinoléines sont des produits connus.

En opérant comme précédemment, on a obtenu les produits (I) à partir des produits de départ (V).

Exemple 49

Chlorhydrate de O-(4-éthoxycarbonylphényl) hydroxylamine

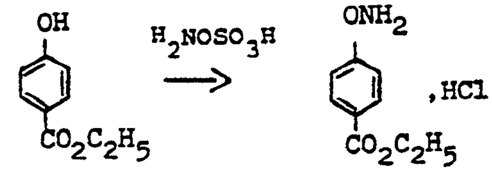


F=135°C

Exemple 50

Chlorhydrate de O-(4-terbutylphényl) hydroxylamine

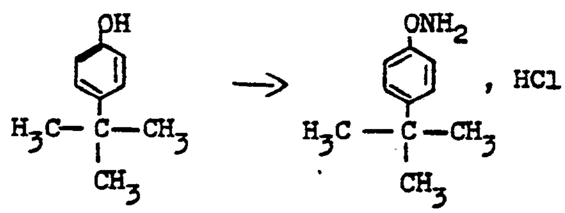

F=140°C

Exemple 51

O-(2,4-dichlorophényl) hydroxylamine

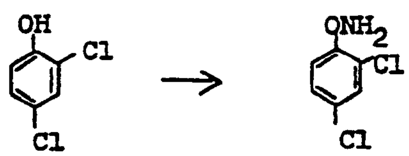

F=79-80°C

Exemple 52

Chlorhydrate de O-(4-fluorophényl) hydroxylamine

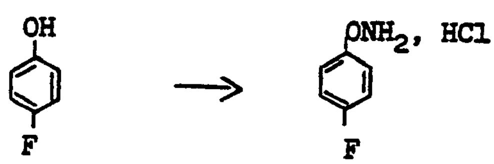

F=144-146°C (décomposition)

Exemple 53

O-(4-diméthoxycarbamoylphényl) hydroxylamine

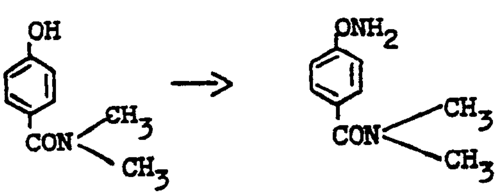

F=103-104°C

Le 4-hydroxy N,N-diméthyl benzamide utilisé comme produit de départ a été préparé selon le schéma suivant:

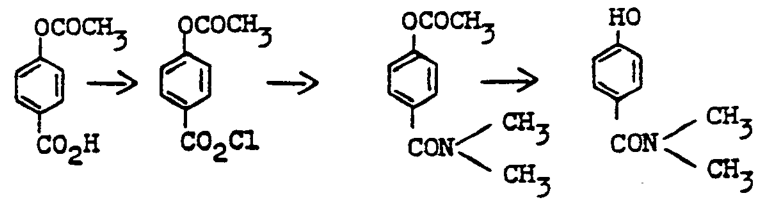

F=160-161°C

Exemple 54

Chlorhydrate de O-(4-chloro 3-méthylphényl) hydroxylamine

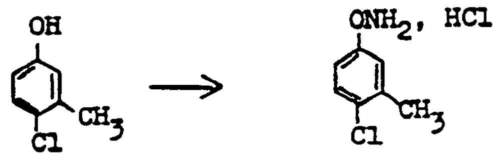

F=154-156°C

Exemple 55

Chlorhydrate de O-(4-chloro 2-méthylphényl) hydroxylamine

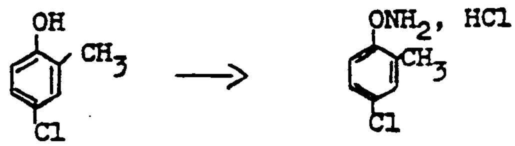

F=130-132°C

Exemple 56

O-(4-cyanophényl) hydroxylamine

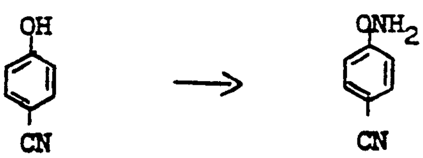

F=110°C

Exemple 57

Chlorhydrate de O-(3-chlorophényl) hydroxylamine

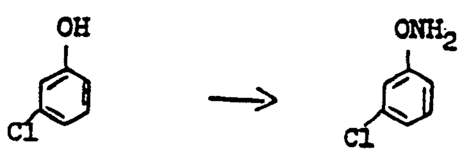

, HCl

F=155°C

Exemple 58

O-(3-nitrophényl) hydroxylamine

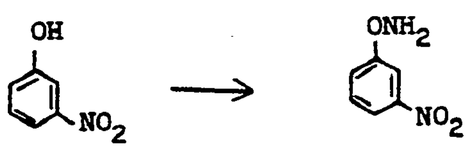

F=56°C

Exemple 59

O-(4-acétylphényl) hydroxylamine

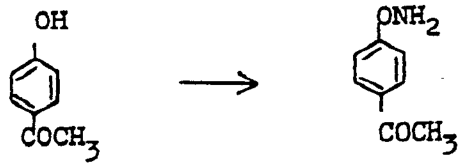

F=118°C

Exemple 60

Chlorhydrate de O-(3,4-dichlorophényl) hydroxylamine

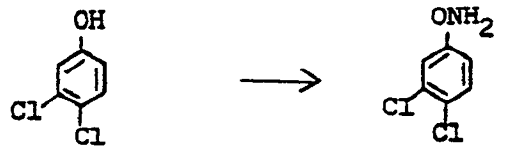

, HCl F=175°C

Ci-après figurent deux exemples de composés de formule (I) pouvant être obtenus selon le procédé de l'invention.

Exemple 61

O-[4-nitro 3-(méthylsulfonylméthyl) phényl] hydroxylamine

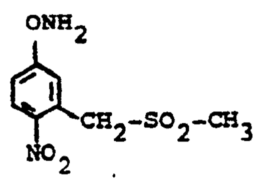

Exemple 62

O-(4-nitro 3-cyanométhylphényl) hydroxylamine

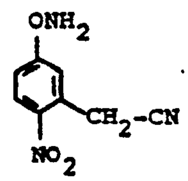

Exemple 63

O-(3-(propyn-2-yloxy) 4-nitrophényl) hydroxylamine

On agite et refroidit à +5°C une solution renfermant 19,5 g de 2-(prop-1-yn-3-yloxy)4-fluoro nitrobenzène, 150 cm3 d'éthanol 50 cm3 d'eau et 8,4 g de chlorhydrate d'hydroxylamine. On introduit, entre 5 et 10°C, en 30 minutes, 22 cm3 de soude 10N. On agite pendant 16 heures à 20°C. On ajoute de l'eau, refroidit à 5°C en agitant. On filtre, lave à l'eau et sèche sous pression réduite. On obtient 15 g de produit recherché fondant à 95 96°C. On recristallise dans le méthanol et obtient 8 g de produit pur fondant à 99—100°C.

Le 2-(prop-1-yn-3-yloxy)4-fluoro nitrobenzène utilisé comme produit de départ de l'exemple 63 a été préparé comme suit:

Stade A: 3-fluoro (prop-1-yn 3-yloxy)benzène

Le produit a été préparé en soumettant le 3-fluorophénol à l'action du bromure de propargyle. Eb. = 95—96°C/20 mm de mercure.

Stade B: 2-(prop-1-yn-3-yloxy)4-fluoronitrobenzène.

Le produit a été préparé selon le procédé de nitration décrit ci-dessus. F = 38—39°C.

Exemples de compositions

*Concentré émulsifiable:*

On a préparé une composition contenant en poids 15% de produit de l'exemple 1, 6,4% Atlox 4851 (triglycéride oxyéthyléné combiné avec un sulfonate, indice d'acide 1,5), 3,2% d'Atlox 4855 (triglycéride oxyéthyléné combiné avec un sulfonate, indice d'acide 3) et 75,4% de xylène.

*Poudre mouillable:*

On a préparé une poudre mouillable contenant 25% de produit de l'exemple 2, 15% d'Ekapersol (produit de condensation du naphtalène sulfonate de sodium), 35% de Zéosil 39 (silice hydratée synthétique obtenue par précipitation), et 25% de Vercoryl S (kaolin colloïdal).

Etude de l'activité des produits de l'invention

A) *Etude de l'effet "Warburg"*

Les produits de l'invention possèdent la propriété de diminuer l'effet inhibiteur de l'oxygène sur la photosynthèse (effet Warburg), ce qui a pour conséquence une stimulation de la photosynthèse et une augmentation du rendement de la récolte.

Le test est effectué sur des feuilles de blé excisées, placées en flottaison soit sur de l'eau distillée, soit sur une solution de produit à étudier (10 mmoles par litre).

Les feuilles sont placées dans des chambres de verre étanches sous un éclairement de 300 W/m2 et dont la température est réglée à 25°C; l'atmosphère de la chambre est continuellement renouvelée soit avec de l'air normal (21% $O_2$ — 350 ppm $CO_2$), soit de l'oxygène pur: près de 100%). Lorsque la photosynthèse stationnaire est atteinte, 0,09 µmole de $^{14}CO_2$ est introduite (activité spécifique: 22 mCi/mM) et les feuilles sont laissées pendant 15 minutes à la lumière en présence de gaz carbonique radioactif.

Les feuilles sont ensuite plongées dans l'azote liquide et conservées. Elles sont ensuite brûlées afin de déterminer la quantité de $^{14}CO_2$ fixée par photosynthèse. Les résultats sont exprimés en rapports de radioactivité fixée entre feuilles témoins et traitées avec les produits à étudier.

Les feuilles ont été traitées avec 10 µmoles de certains produits des exemples et sont placées dans une atmosphère contenant 100% d'$O_2$.

Les résultats obtenus sur l'inhibition de l'oxygène vis-à-vis de la photosynthèse sont les suivants:

| | Photosynthèse en % du témoin non traité (100) |
|---|---|
| Produit de l'exemple 1 | 165 (±15) |
| Produit de l'exemple 2 | 140 (±13) |
| Produit de l'exemple 3 | 138 (±12) |
| Produit de l'exemple 4 | 145 (±10) |
| Produit de l'exemple 5 | 125 (±9) |
| Produit de l'exemple 33 | 140 (±11) |
| Produit de l'exemple 51 | 152 (±12) |
| Produit de l'exemple 57 | 160 (±14) |

Le tableau exprime le pourcentage de la photosynthèse de feuilles de blé, mesurée par absorption du $CO_2$ par rapport au témoin non traité en présence d'une atmosphère contenant 100% d'oxygène (l'inhibition de la photosynthèse par l'$O_2$ est de 30 à 50% dans ces essais).

B) *Etude de l'activité comme facteur de croissance des produits des exemples 1 et 2*

Test sur tomates: Le test est effectué sur une culture de tomates de variété Europcel. On utilise un bloc de Fisher à 5 répétitions. Les parcelles élémentaires sont de 20 m2 (10 × 2 m) et un témoin non traité est inclus dans chaque répétition. L'essai est implanté sur un sol argileux, calcaro-limoneux. Les traitements sont réalisés avec un appareil à dos du type Van de Weij sur la base de 750 l/ha sous une pression constante de 3 bars. Les traitements sont effectués soit après la chute des pétales des premières fleurs ($T_1$), soit après la chute des pétales des dernières fleurs ($T_2 = T_1 + 20$ jours), soit à la nouaison, c'est-à-dire au moment de la formation des fruits ($T_3 = T_2 + 20$ jours). Les produits des exemples 1 et 2 sont utilisés à des doses de 40, 60 ou 120 g/ha en 3 fois ou 1 fois. Deux récoltes de tomates sont réalisées. A chaque récolte, on détermine le rendement en kg pour 32 plants de tomates, par rapport à un témoin non traité.

On a trouvé que sur ce test, les produits des exemples 1 et 2 présentaient une activité de facteur de croissance intéressante.

**Revendications pour les Etats contractants: BE CH DE FR GB IT LI LU NL SE**

1. Les composés de formule (I)

$$Ar—O—NH_2 \qquad (I)$$

dans laquelle Ar représente un radical aromatique ou hétéroaromatique, mono ou polycyclique, éventuellement substitué par un ou plusieurs radicaux choisis dans le groupe constitué par le radical OH, les atomes d'halogène, les groupements $NO_2$ et CN, les radicaux

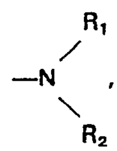 , 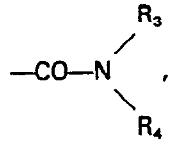 , 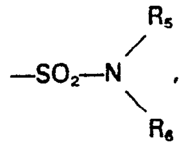 ,

dans lesquels $R_1$, $R_2$, $R_3$, $R_4$, $R_5$ et $R_6$, identiques ou différents, représentent un atome d'hydrogène ou un radical alcoyle linéaire ou ramifié renfermant de 1 à 8 atomes de carbone; les radicaux $R_7$ et $OR_8$, $R_7$ et $R_8$ représentant des radicaux alcoyles linéaires ou ramifiés, saturés ou insaturés renfermant jusqu'à 8 atomes de carbone, éventuellement substitués par un ou plusieurs atomes d'halogène ou par un radical cyano, les radicaux —CO $R_9$, les radicaux

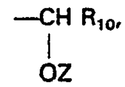

Z représentant un atome d'hydrogène, un radical alcoyle, linéaire où ramifié, saturé ou insaturé, renfermant jusque'à 8 atomes de carbone ou un radical acyle renfermant de 2 à 18 atomes de carbone, les radicaux $NHCOR_{11}$, $SO_2$ $R_{12}$, $SO_3$ $R_{13}$ dans lesquels $R_9$, $R_{10}$, $R_{11}$, $R_{12}$ et $R_{13}$ représentant un radical alcoyle renfermant de 1 à 8 atomes de carbone, les radicaux $CO_2$ $R_{14}$ dans lesquels $R_{14}$ représente un atome d'hydrogène ou un radical alcoyle renfermant de 1 à 8 atomes de carbone, les radicaux aryle renfermant de 6 à 14 atomes de carbone, les groupements $—CH_2—CN$ ou $—CH_2—SO_2—R_{15}$ dans lesquels $R_{15}$ représente un radical alcoyle renfermant de 1 à 8 atomes de carbone ou un radical aryle renfermant de 6 à 14 atomes de carbone éventuellement substitué par un radical alcoyle renfermant de 1 à 8 atomes de carbone et les groupements $—OR_{16}$ dans lesquels $R_{16}$ représente un radical aryle renfermant de 6 à 14 atomes de carbone, éventuellement substitué par un radical alcoyle renfermant de 1 à 8 atomes de carbone, par un atome d'halogène ou par un radical $NO_2$, les substituants de Ar pouvant éventuellement former des cycles renfermant éventuellement un ou plusieurs hétéroatomes choisis parmi l'oxygène, l'azote et le soufre, ainsi que leurs sels d'addition avec les acides, à la condition que Ar ne représente ni un radical phényle non substitué, ni un radical phényle monosubstitué par un radical méthyle en 2, 3 ou 4, par un radical nitro en 2 ou 4, par un atome de chlore en 3 ou 4, par un atome de brome en 4 ou par un radical $CF_3$ en 3 ou 4, ni un radical phényle ou bien disubstitué en 2 par un radical nitro et en 4 par un radical $CF_3$ ou par un radical nitro, ou bien disubstitué en 2 et 6 par un radical nitro, ni un radical phényle trisubstitué par des radicaux nitro en 2, 4 et 6 ou par des radicaux nitro en 2 et 4 et par un radical $CF_3$ en 6.

2. Les composés de formule (I) tels que définis à la revendication 1, pour lesquels Ar représente un radical phényle substitué, ainsi que leurs sels d'addition avec les acides.

3. Les composés de formule (I) tels que définis à la revendication 2 dans lesquels l'un au moins des substituants du radical phényle est un radical nitro.

4. Les composés de formule (I) tels que définis à la revendication 2 dans lesquels l'un au moins des substituants du radical phényle est un atome de chlore en para.

5. Les composés de formule (I) tels que définis à la revendication 3 ou 4 pour lesquels Ar représente un radical phényle substitué en 2 par un radical nitro et en 4 par un atome de chlore.

6. Les composés de formule (I) tels que définis à la revendication 3 pour lesquels Ar représente un radical phényle substitué en 4 par un radical nitro et en 3 par un radical alcoxyle renfermant de 1 à 8 atomes de carbone.

7. Les composés de formule (I) tels que définis à la revendication 3 pour lesquels Ar représente un radical phényle substitué en 4 par un radical nitro et en 2 par un radical alcoyle linéaire ou ramifié renfermant de 1 à 8 atomes de carbone.

8. Les composés de formule (I) tels que définis à la revendication 1 pour lesquels Ar représente le radical:

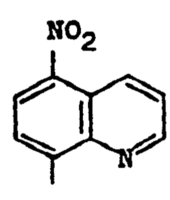

éventuellement substitué par un ou plusieurs des substituants mentionnés dans la revendication 1.

9. Les composés de formule (I) tels que définis à la revendication 1, pour lesquels Ar représente le radical:

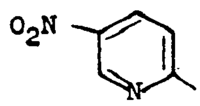

10. L'un quelconque des composés de formule (I) tels que définis à la revendication 1 dont les noms suivent:

— l'O-(2-méthyl 4-nitrophényl) hydroxylamine,
— l'O-(4-nitro 3-méthoxyphényl) hydroxylamine,

ainsi que leurs sels d'addition avec les acides.

11. L'un quelconque des composés de formule (I) tels que définie à la revendication 1, dont les noms suivent:

— l'O-(5-nitro 8-quinoléyl) hydroxylamine,
— l'O-(2-chloro 4-nitrophényl) hydroxylamine,
— l'O-(2-nitro 4-chlorophényl) hydroxylamine,
— l'O-(2,4-dichlorophényl) hydroxylamine,
— l'O-(4-nitro 2-trifluorométhylphényl) hydroxylamine,
— l'O-[2-(1RS-hydroxyéthyl) 4-nitrophényl] hydroxylamine,
— l'O-(4-cyanophényl) hydroxylamine,
— l'O-[3-(propyn-2-yloxy) 4-nitrophényl] hydroxylamine,
— l'O-(3,4-dichlorophényl) hydroxylamine,

ainsi que leurs sels d'addition avec les acides.

12. Les composés de formule (I), tels que définis à l'une quelconque des revendications 1 à 11, ainsi que leurs sels d'addition avec les acides, pour leur utilisation comme facteurs de croissance des végétaux.

13. Les compositions destinées à l'usage agricole pour être utilisées comme facteurs de croissance des végétaux, caractérisées en ce qu'elles renferment comme principe actif au moins un composé de formule (I) tel que défini à l'une quelconque des revendications 1 à 9, ainsi que leurs sels d'addition avec les acides.

14. Les compositions destinées à l'usage agricole pour être utilisées comme facteurs de croissance des végétaux, caractérisées en ce qu'elles renferment comme principe actif au moins un composé défini à la revendication 10.

15. Les compositions destinées à l'usage agricole pour être utilisées comme facteurs de croissance des végétaux, caractérisées en ce qu'elles renferment comme principe actif au moins un composé défini à la revendication 11.

16. Procédé de préparation des composés de formule (I) et de leurs sels, tels que définis à l'une quelconque des revendications 1 à 11, caractérisé en ce que l'on soumet un composé de formule:

Ar—Hal (II)

dans laquelle Ar conserve la même signification que précédemment et Hal représente un atome d'halogène, à l'action d'un composé de formule:

$$HO-N(R)(R) \quad (III)$$

dans laquelle R représente un atome d'hydrogène ou les deux radicaux R forment un reste phtalimido, pour obtenir un composé de formule:

$$Ar-O-N(R)(R) \quad (IV)$$

dans laquelle Ar et R ont la signification précitée, correspondant à un composé de formule (I), dans le cas où R représente un atome d'hydrogène, composé de formule (IV) que l'on fait réagir lorsque R est différent d'hydrogène avec l'hydrazine, pour obtenir un composé de formule (I), puis fait réagir, si désiré, les composés de formule (I) obtenus avec un acide, pour en former les sels.

17. Procédé de préparation des composés de formule (I) et de leurs sels, tels que définis à l'une quelconque des revendications 1 à 11, caractérisé en ce que l'on soumet un composé de formule:

$$Ar—OH \qquad (V)$$

dans laquelle Ar garde sa signification précédente, à l'action d'un composé de formule:

$$H_2NOSO_3H \qquad (VI)$$

pour obtenir le composé de formule (I) correspondant que l'on fait réagir, si désiré, avec un acide pour en former le sel.

**Revendications pour l'Etat contractant: AT**

1. Procédé pour préparer les composés de formule (I):

$$Ar—O—NH_2 \qquad (I)$$

dans laquelle Ar représente un radical aromatique ou hétéroaromatique, mono ou polycyclique, éventuellement substitué par un ou plusieurs radicaux choisis dans le groupe constitué par le radical OH, les atomes d'halogène, les groupements $NO_2$ et CN, les radicaux

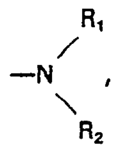 , 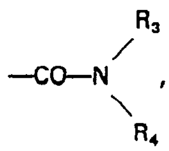 , 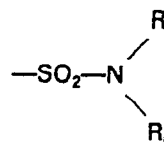 ,

dans lesquels $R_1$, $R_2$, $R_3$, $R_4$, $R_5$ et $R_6$, identiques ou différents, représentent un atome d'hydrogène ou un radical alcoyle linéaire ou ramifié renfermant de 1 à 8 atomes de carbone; les radicaux $R_7$ et $OR_8$, $R_7$ et $R_8$ représentant des radicaux alcoyles linéaires ou ramifiés, saturés ou insaturés renfermant jusqu'à 8 atomes de carbone, éventuellement substitués par un ou plusieurs atomes d'halogène ou par un radical cyano, les radicaux —CO $R_9$, les radicaux

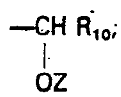;

Z représentant un atome d'hydrogène, un radical alcoyle, linéaire ou ramifié, saturé ou insaturé, renfermant jusque'à 8 atomes de carbone ou un radical acyle renfermant de 2 à 18 atomes de carbone, les radicaux $NHCOR_{11}$, $SO_2$ $R_{12}$, $SO_3$ $R_{13}$ dans lesquels $R_9$, $R_{10}$, $R_{11}$, $R_{12}$ et $R_{13}$ représentant un radical alcoyle renfermant de 1 à 8 atomes de carbone, les radicaux $CO_2$ $R_{14}$ dans lesquels $R_{14}$ représente un atome d'hydrogène ou un radical alcoyle renfermant de 1 à 8 atomes de carbone, les radicaux aryle renfermant de 6 à 14 atomes de carbone, les groupements $—CH_2—CN$ ou $—CH_2—SO_2—R_{15}$ dans lesquels $R_{15}$ représente un radical alcoyle renfermant de 1 à 8 atomes de carbone ou un radical aryle renfermant de 6 à 14 atomes de carbone éventuellement substitué par un radical alcoyle renfermant de 1 à 8 atomes de carbone et les groupements $—OR_{16}$ dans lesquels $R_{16}$ représente un radical aryle renfermant de 6 à 14 atomes de carbone, éventuellement substitué par un radical alcoyle renfermant de 1 à 8 atomes de carbone, par un atome d'halogène ou par un radical $NO_2$, les substituants de Ar pouvant éventuellement former des cycles renfermant éventuellement un ou plusieurs hétéroatomes choisis parmi l'oxygène, l'azote et le soufre, ainsi que leurs sels d'addition avec les acides, à la condition que Ar ne représente ni un radical phényle non substitué, ni un radical phényle monosubstitué par un radical méthyle en 2, 3 ou 4, par un radical nitro en 2 ou 4, par un atome de chlore en 3 ou 4, par un atome de brome en 4 ou par un radical $CF_3$ en 3 ou 4, ni un radical phényle ou bien disubstitué en 2 par un radical nitro et en 4 par un radical $CF_3$ ou par un radical nitro, ou bien disubstitué en 2 et 6 par un radical nitro, ni un radical phényle trisubstitué par des radicaux nitro en 2, 4 et 6 ou par des radicaux nitro en 2 et 4 et par un radical $CF_3$ en 6, caractérisé en ce que l'on soumet un composé de formule:

$$Ar—Hal \qquad (II)$$

dans laquelle Ar conserve la même signification que précédemment et Hal représente un atome d'halogène, à l'action d'un composé de formule:

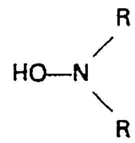

(III)

dans laquelle R représente un atome d'hydrogène ou les deux radicaux R forment un reste phtalimido, pour obtenir un composé de formule:

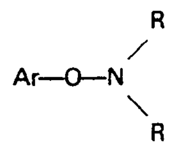

(IV)

dans laquelle Ar et R ont la signification précitée, correspondant à un composé de formule (I), dans le cas où R représente un atome d'hydrogène, composé de formule (IV) que l'on fait réagir lorsque R est différent d'hydrogène avec l'hydrazine, pour obtenir un composé de formule (I), puis fait réagir, si désiré, les composés de formule (I) obtenus avec un acide, pour en former les sels.

2. Procédé selon la revendication 1, caractérisé en ce que

— dans le cas où, dans la formule (III), R représente un atome d'hydrogène, on utilise un produit de formule (II) dans laquelle Hal représente un atome de fluor ou de brome;

— on fait réagir, les cas échéant, un sel d'hydroxylamine, par exemple le chlorhydrate, sur le composé Ar—Hal en solution dans un alcool ou un milieu hydroalcoolique et en présence d'une base forte comme la soude, le potasse ou un alcoolate;

— on fait réagir, le cas échéant, le composé de formule (III) dans laquelle les radicaux R forment un reste phtalimido, avec un base forte comme l'hydrure de sodium ou de potassium puis avec le composé de formule (II), ou encore directement avec le composé de formule (II) au sein d'un solvant inerte, comme par exemple l'acétonitrile en présence d'une base tertiaire comme la triéthylamine ou la pyridine.

3. Procédé de préparation des composés de formule (I) et de leurs sels, tels que définis à la revendication 1, caractérisé en ce que l'on soumet un composé de formule:

$$Ar{-}OH \qquad (V)$$

dans laquelle Ar garde sa signification précédente, à l'action d'un composé de formule:

$$H_2NOSO_3H \qquad (VI)$$

pour obtenir le composé de formule (I) correspondant que l'on fait réagir, si désiré, avec un acide pour en former le sel.

4. Procédé selon l'une quelconque des revendications 1 à 3, pour la préparation des composés de formule (I), tels que définis à la revendication 1, répondant à la formule ($I_A$):

$$Ar'{-}O{-}NH_2 \qquad (I_A)$$

dans laquelle Ar' représente un radical aromatique ou hétéroaromatique, mono ou polycyclique, éventuellement substitué par un ou plusieurs radicaux choisis dans le groupe constitué par le radical OH, les atomes d'halogène, les groupements $NO_2$ et CN, les radicaux

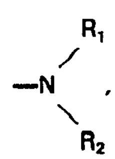 , 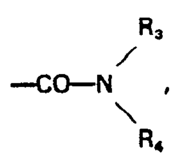 , 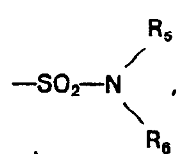 ,

dans lesquels $R_1$, $R_2$, $R_3$, $R_4$, $R_5$ et $R_6$, identiques ou différents, représentent un atome d'hydrogène ou un radical alcoyle linéaire ou ramifié renfermant de 1 à 8 atomes de carbone; les radicaux $R_7$ et $OR_8$, $R_7$ et $R_8$ représentant des radicaux alcoyles linéaires ou ramifiés, saturés ou insaturés renfermant jusqu'à 8 atomes de carbone, éventuellement substitués par un ou plusieurs atomes d'halogène ou par un radical cyano, les radicaux —CO $R_9$, les radicaux

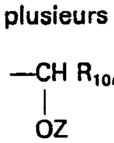

Z représentant un atome d'hydrogène, un radical alcoyle, linéaire ou ramifié, saturé ou insaturé,

renfermant jusque'à 8 atomes de carbone ou un radical acyle renfermant de 2 à 18 atomes de carbone, les radicaux $NHCOR_{11}$, $SO_2$ $R_{12}$, $SO_3$ $R_{13}$ dans lesquels $R_9$, $R_{10}$, $R_{11}$, $R_{12}$ et $R_{13}$ représentant un radical alcoyle renfermant de 1 à 8 atomes de carbone, les radicaux $CO_2$ $R_{14}$ dans lesquels $R_{14}$ représente un atome d'hydrogène ou un radical alcoyle renfermant de 1 à 8 atomes de carbone et les radicaux aryle renfermant 6 à 14 atomes de carbone, les substituants de Ar' pouvant éventuellement former des cycles renfermant éventuellement un plusieurs hétéroatomes choisis parmi l'oxygène, l'azote et le soufre, ainsi que leurs sels d'addition avec les acides, à la condition que Ar' ne représente ni un radical phényle non substitué, ni un radical phényle monosubstitué par un radical méthyle en 2, 3 ou 4, par un radical nitro en 2 ou 4, par un atome de chlore en 3 ou 4, par un atome de brome en 4 ou par un radical $CF_3$ en 3 ou 4, ni un radical phényle ou bien disubstitué en 2 par un radical nitro et en 4 par un radical $CF_3$ ou par un radical nitro ou bien disubstitué en 2 et 6 par un radical nitro, ni un radical phényle trisubstitué par des radicaux nitro en 2, 4 et 6 ou par des radicaux nitro en 2 et 4 et par un radical $CF_3$ en 6, caractérisé en ce que l'on utilise au départ un composé de formule (II) ou (V) dans laquelle Ar a les significations de Ar' ci-dessus.

5. Procédé selon la revendication 4, caractérisé en ce que l'on utilise au départ un composé de formule (II) ou (V) dans laquelle Ar représente un radical phényle substitué.

6. Procédé selon la revendication 4 ou 5, caractérisé en ce que l'on utilise au départ un composé de formule (II) ou (V) dans laquelle Ar représente un radical phényle substitué au moins par un radical nitro.

7. Procédé selon la revendication 4 ou 5, caractérisé en ce que l'on utilise au départ un composé de formule (II) ou (V) dans laquelle Ar représente un radical phényle substitué au moins par un atome de chlore en position para.

8. Procédé selon la revendication 5, 6 ou 7, caractérisé en ce que l'on utilise au départ un composé de formule (II) ou (V) dans laquelle Ar représente un radical phényle substitué en 2 par un radical nitro et en 4 par un atome de chlore.

9. Procédé selon la revendication 5 ou 6, caractérisé en ce que l'on utilise au départ un composé de formule (II) ou (V) dans laquelle Ar représente un radical phényle substitué en 4 par un radical nitro et en 3 par un radical alcoxyle renfermant de 1 à 8 atomes de carbone.

10. Procédé selon la revendication 5 ou 6, caractérisé en ce que l'on utilise au départ un composé de formule (II) ou (V) dans laquelle Ar représente un radical phényle substitué en 4 par un radical nitro et en 2 par un radical alcoyle linéaire ou ramifié renfermant de 1 à 8 atomes de carbone.

11. Procédé selon la revendication 4, caractérisé en ce que l'on utilise au départ un composé de formule (II) ou (V) dans laquelle Ar représente un radical

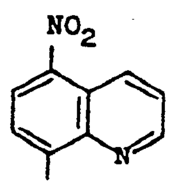

éventuellement substitué.

12. Procédé selon la revendication 4, caractérisé en ce que l'on utilise au départ un composé de formule (II) ou (V) dans laquelle Ar représente un radical

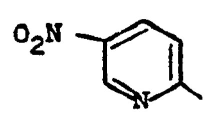

13. Procédé selon la revendication 4, caractérisé en ce que l'on utilise au départ un composé de formule (II) ou (V) dans laquelle Ar représente un radical 2-méthyl 4-nitrophényl ou un radical 3-méthoxy 4-nitrophényl.

14. Procédé selon la revendication 4, caractérisé en ce que l'on utilise au départ un composé de formule (II) ou (V) dans laquelle Ar représente un radical choisi dans le groupe formé par les radicaux:

— 5-nitro 8-quinoléyl;
— 2-chloro 4-nitrophényl;
— 2-nitro 4-chlorophényl;
— 2,4-dichlorophényl;
— 4-nitro 2-trifluorométhylphényl;
— 2-(1-(RS)-hydroxyéthyl) 4-nitrophényl;
— 4-cyanophényl;
— 3-(propyn-2-yloxy) 4-nitrophényl et
— 3,4-dichlorophényl.

15. Les compositions destinées à l'usage agricole pour être utilisées comme facteurs de croissance des végétaux, caractérisées en ce qu'elles renferment comme principe actif au moins un composé de formule (I), tel que défini à la revendication 1, ainsi que leurs sels d'addition avec les acides.

16. Les compositions destinées à l'usage agricole pour être utilisées comme facteurs de croissance des végétaux, caractérisées en ce qu'elles renferment comme principe actif au moins un composé de formule (I), tel que défini à l'une quelconque des revendications 4 à 12, ainsi que leurs sels d'addition avec les acides.

17. Les compositions destinées à l'usage agricole pour être utilisées comme facteurs de croissance des végétaux, caractérisées en ce qu'elles renferment comme principe actif au moins un composé défini à la revendication 13.

18. Les compositions destinées à l'usage agricole pour être utilisées comme facteurs de croissance de végétaux, caractérisées en ce qu'elles renferment comme principe actif au moins un composé défini à la revendication 14.

**Patentansprüche für die Vertragsstaaten: BE CH DE FR GB IT LI LU NL SE**

1. Verbindungen der Formel (I)

$$Ar{-}O{-}NH_2 \qquad (I)$$

worin Ar einen mono- oder polycyclischen, aromatischen oder heteroaromatischen Rest bedeutet, der gegebenenfalls substituiert ist durch einen oder mehrere Reste, ausgewählt unter der OH-Gruppe, den Halogenatomen, den $NO_2$- und CN-Gruppen, den Resten

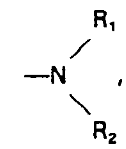 , 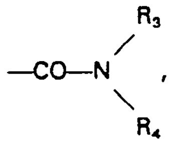 , 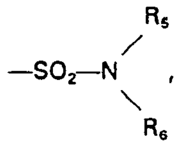 ,

worin $R_1$, $R_2$, $R_3$, $R_4$, $R_5$ und $R_6$, die gleich oder verschieden sind, ein Wasserstoffatom oder einen linearen oder verzweigten Alkylrest mit 1 bis 8 Kohlenstoffatomen bedeuten; den Resten $R_7$ und $OR_8$, wobei $R_7$ und $R_8$ gesättigte oder ungesättigte, lineare oder verzweigte Alkylreste mit bis zu 8 Kohlenstoffatomen, die gegebenenfalls durch ein oder mehrere Halogenatome oder durch einen Cyanorest substituiert sind, bedeuten; den Resten —CO—$R_9$, den Resten

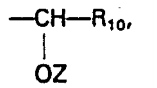

worin Z ein Wasserstoffatom, einen gesättigten oder ungesättigten, linearen oder verzweigten Alkylrest mit bis zu 8 Kohlenstoffatomen oder einen Acylrest mit 2 bis 18 Kohlenstoffatomen bedeutet, den Resten $NHCOR_{11}$, $SO_2$—$R_{12}$, $SO_3$—$R_{13}$, worin $R_9$, $R_{10}$, $R_{11}$, $R_{12}$ und $R_{13}$ einen Alkylrest mit 1 bis 8 Kohlenstoffatomen bedeuten, den Resten $CO_2$—$R_{14}$, worin $R_{14}$ ein Wasserstoffatom oder einen Alkylrest mit 1 bis 8 Kohlenstoffatomen bedeutet, den Arylresten mit 6 bis 14 Kohlenstoffatomen, den Gruppen —$CH_2$—CN oder —$CH_2$—$SO_2$—$R_{15}$, worin $R_{15}$ einen Alkylrest mit 1 bis 8 Kohlenstoffatomen oder einen Arylrest mit 6 bis 14 Kohlenstoffatomen, der gegebenenfalls durch einen Alkylrest mit 1 bis 8 Kohlenstoffatomen substituiert ist, bedeutet, und den Gruppen —$OR_{16}$, worin $R_{16}$ einen Arylrest mit 6 bis 14 Kohlenstoffatomen bedeutet, der gegebenenfalls durch einen Alkylrest mit 1 bis 6 Kohlenstoffatomen, durch ein Halogenatom oder durch eine $NO_2$-Gruppe substituiert ist, wobei die Substituenten von Ar gegebenenfalls Ringe bilden können, die gegebenenfalls ein oder mehrere Heteroatome, ausgewählt unter Sauerstoff, Stickstoff und Schwefel, enthalten, sowie deren Additionssalze mit Säuren, unter der Voraussetzung, daß Ar weder einen Phenylrest, der unsubstituiert ist, noch einen Phenylrest, der durch eine Methylgruppe in 2-, 3- oder 4-Stellung, durch eine Nitrogruppe in 2- oder 4-Stellung, durch ein Chloratom in 3- oder 4-Stellung, durch ein Bromatom in 4-Stellung oder durch eine $CF_3$-Gruppe in 3- oder 4-Stellung monosubstituiert ist, noch einen Phenylrest, der entweder in 2-Stellung durch eine Nitrogruppe und in 4-Stellung durch eine $CF_3$-Gruppe oder durch eine Nitrogruppe disubstituiert oder in 2- und 6-Stellung durch eine Nitrogruppe disubstituiert ist, noch einen Phenylrest, der durch Nitrogruppen in 2-, 4- und 6-Stellung oder durch Nitrogruppen in 2- und 4-Stellung und durch eine $CF_3$-Gruppe in 6-Stellung trisubstituiert ist, bedeutet.

2. Verbindungen der Formel (I) gemäß Anspruch 1, worin Ar einen substituierten Phenylrest bedeutet, sowie deren Additionssalze mit Säuren.

3. Verbindungen der Formel (I) gemäß Anspruch 2, worin zumindest einer der Substituenten des Phenylrestes eine Nitrogruppe ist.

4. Verbindungen der Formel (I) gemäß Anspruch 2, worin zumindest einer der Substituenten des Phenylrestes ein Chloratom in para-Stellung ist.

5. Verbindungen der Formel (I) gemäß Anspruch 3 oder 4, worin Ar einen in 2-Stellung durch eine Nitrogruppe und in 4-Stellung durch ein Chloratom substituierten Phenylrest bedeutet.

6. Verbindungen der Formel (I) gemäß Anspruch 3, worin Ar einen in 4-Stellung durch eine Nitrogruppe und in 3-Stellung durch eine Alkoxygruppe mit 1 bis 8 Kohlenstoffatomen substituierten Phenylrest bedeutet.

7. Verbindungen der Formel (I) gemäß Anspruch 3, worin Ar einen in 4-Stellung durch eine Nitrogruppe und in 2-Stellung durch einen linearen oder verzweigten Alkylrest mit 1 bis 8 Kohlenstoffatomen substituierten Phenylrest bedeutet.

8. Verbindungen der Formel (I) gemäß Anspruch 1, worin Ar den Rest

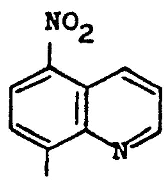

bedeutet, der gegebenenfalls durch einen oder mehrere der in Anspruch 1 genannten Substituenten substituiert ist.

9. Verbindungen der Formel (I) gemäß Anspruch 1, worin Ar den Rest

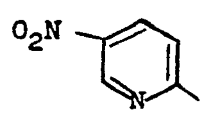

bedeutet.

10. Eine der Verbindungen der Formel (I) gemäß Anspruch 1, mit den folgenden Bezeichnungen:
O-(2-Methyl 4-nitrophenyl)-hydroxylamin,
O-(4-Nitro-3-methoxyphenyl)-hydroxylamin
sowie deren Additionssalze mit Säuren.

11. Eine der Verbindungen der Formel (I) gemäß Anspruch 1, mit den folgenden Bezeichnungen:
O-(5-Nitro-8-chinolyl)-hydroxylamin,
O-(2-Chlor-4-nitrophenyl)-hydroxylamin,
O-(2-Nitro-4-chlorphenyl)-hydroxylamin,
O-(2,4-Dichlorphenyl)-hydroxylamin,
O-(4-Nitro-2-trifluormethylphenyl)-hydroxylamin,
O-[2-(1RS-Hydroxyethyl)-4-nitrophenyl]-hydroxylamin,
O-(4-Cyanophenyl)-hydroxylamin,
O-[3-(Propin-2-yloxy)-4-nitrophenyl]-hydroxylamin,
O-(3,4-Dichlorphenyl)-hydroxylamin
sowie deren Additionssalze mit Säuren.

12. Verbindungen der Formel (I) gemäß einem der Ansprüche 1 bis 11 sowie deren Additionssalze mit Säuren zu deren Verwendung als Wachstumsfaktoren für Pflanzen.

13. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung der Formel (I) gemäß einem der Ansprüche 1 bis 9 sowie deren Additionssalze mit Säuren enthalten.

14. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung gemäß Anspruch 10 enthalten.

15. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung gemäß Anspruch 11 enthalten.

16. Verfahren zur Herstellung der Verbindungen der Formel (I) und von deren Salzen gemäß einem der Ansprüche 1 bis 11, dadurch gekennzeichnet, daß man eine Verbindung der Formel

$$Ar—Hal \qquad (II)$$

worin Ar die vorstehend angegebene Bedeutung besitzt und Hal ein Halogenatom bedeutet, der Einwirkung einer Verbindung der Formel

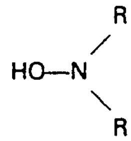

(III)

worin R ein Wasserstoffatom bedeutet oder die beiden Reste R einen Phthalimidorest bilden, unterzieht, um eine Verbindung der Formel

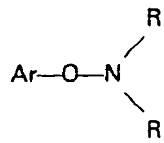

(IV)

worin Ar und R die vorstehende Bedeutung besitzen, entsprechend einer Verbindung der Formel (I), worin R ein Wasserstoffatom bedeutet, zu erhalten, die Verbindung der Formel (IV), wenn R von Wasserstoff verschieden ist, mit Hydrazin umsetzt, um eine Verbindung der Formel (I) zu erhalten, danach gewünschtenfalls die erhaltenen Verbindungen der Formel (I) mit einer Säure umsetzt, um hieraus das Salz zu bilden.

17. Verfahren zur Herstellung der Verbindungen der Formel (I) und von deren Salzen gemäß einem der Ansprüche 1 bis 11, dadurch gekennzeichnet, daß man eine Verbindung der Formel

$$Ar{-}OH \qquad (V)$$

worin Ar die vorstehende Bedeutung besitzt, der Einwirkung einer Verbindung der Formel

$$H_2NOSO_3H \qquad (VI)$$

unterzieht, um die entsprechende Verbindung der Formel (I) zu erhalten, die man gewünschtenfalls mit einer Säure umsetzt, um hieraus das Salz zu bilden.

**Patentansprüche für den Vertragsstaat: AT**

1. Verfahren zur Herstellung der Verbindungen der Formel (I)

$$Ar{-}O{-}NH_2 \qquad (I)$$

worin Ar einen mono- oder polycyclischen, aromatischen oder heteroaromatischen Rest bedeutet, der gegebenenfalls substituiert ist durch einen oder mehrere Reste, ausgewählt unter der OH-Gruppe, den Halogenatomen, den $NO_2$- und CN-Gruppen, den Resten

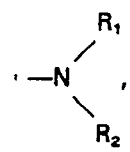 , 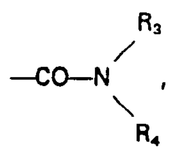 , 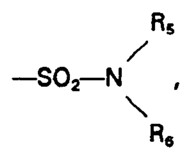 ,

worin $R_1$, $R_2$, $R_3$, $R_4$, $R_5$ und $R_6$, die gleich oder verschieden sind, ein Wasserstoffatom oder einen linearen oder verzweigten Alkylrest mit 1 bis 8 Kohlenstoffatomen bedeuten; den Resten $R_7$ und $OR_8$, wobei $R_7$ und $R_8$ gesättigte oder ungesättigte, lineare oder verzweigte Alkylreste mit bis zu 8 Kohlenstoffatomen, die gegebenenfalls durch ein oder mehrere Halogenatome oder durch einen Cyanorest substituiert sind, bedeuten; den Resten $-CO-R_9$, den Resten

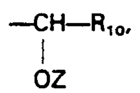

worin Z ein Wasserstoffatom, einen gesättigten oder ungesättigten, linearen oder verzweigten Alkylrest mit bis zu 8 Kohlenstoffatomen oder einen Acylrest mit 2 bis 18 Kohlenstoffatomen bedeutet, den Resten $NHCOR_{11}$, $SO_2-R_{12}$, $SO_3-R_{13}$, worin $R_9$, $R_{10}$, $R_{11}$, $R_{12}$ und $R_{13}$ einen Alkylrest mit 1 bis 8 Kohlenstoffatomen bedeuten, den Resten $CO_2-R_{14}$, worin $R_{14}$ ein Wasserstoffatom oder einen Alkylrest mit 1 bis 8 Kohlenstoffatomen bedeutet, den Arylresten mit 6 bis 14 Kohlenstoffatomen, den Gruppen $-CH_2-CN$ oder $-CH_2-SO_2-R_{15}$, worin $R_{15}$ einen Alkylrest mit 1 bis 8 Kohlenstoffatomen oder einen Arylrest mit 6 bis 14 Kohlenstoffatomen, der gegebenenfalls durch einen Alkylrest mit 1 bis 8 Kohlenstoffatomen

substituiert ist, bedeutet, und den Gruppen $—OR_{16}$, worin $R_{16}$ einen Arylrest mit 6 bis 14 Kohlenstoffatomen bedeutet, der gegebenenfalls durch einen Alkylrest mit 1 bis 8 Kohlenstoffatomen, durch ein Halogenatom oder durch eine $NO_2$-Gruppe substituiert ist, wobei die Substituenten von Ar gegebenenfalls Ringe bilden können, die gegebenenfalls ein oder mehrere Heteroatome, ausgewählt unter Sauerstoff, Stickstoff und Schwefel, enthalten, sowie von deren Additionssalzen mit Säuren, unter der Voraussetzung, daß Ar weder einen Phenylrest, der unsubstituiert ist, noch einen Phenylrest, der durch eine Methylgruppe in 2-, 3- oder 4-Stellung, durch eine Nitrogruppe in 2- oder 4-Stellung, durch ein Chloratom in 3- oder 4-Stellung, durch ein Bromatom in 4-Stellung oder durch eine $CF_3$-Gruppe in 3- oder 4-Stellung monosubstituiert ist, noch einen Phenylrest, der entweder in 2-Stellung durch eine Nitrogruppe und in 4-Stellung durch eine $CF_3$-Gruppe oder durch eine Nitrogruppe disubstituiert oder in 2- und 6-Stellung durch eine Nitrogruppe disubstituiert ist, noch einen Phenylrest, der durch Nitrogruppen in 2-, 4- und 6-Stellung oder durch Nitrogruppen in 2- und 4-Stellung und durch eine $CF_3$-Gruppe in 6-Stellung trisubstituiert ist, bedeutet, dadurch gekennzeichnet, daß man eine Verbindung der Formel

$$Ar—Hal \quad (II)$$

worin Ar die vorstehend angegebene Bedeutung besitzt und Hal ein Halogenatom bedeutet, der Einwirkung einer Verbindung der Formel

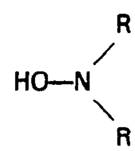
(III)

unterzieht, worin R ein Wasserstoffatom bedeutet oder die beiden Reste R einen Phthalimidorest bilden, um eine Verbindung der Formel

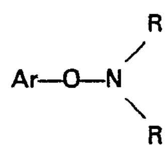
(IV)

worin Ar und R die vorstehende angegebene Bedeutung besitzen, entsprechend einer Verbindung der Formel (I), worin R ein Wasserstoffatom bedeutet, zur erhalten, die Verbindung der Formel (IV), wenn R von Wasserstoff verschieden ist, mit Hydrazin umsetzt, um eine Verbindung der Formel (I) zu erhalten, und danach gewünschtenfalls die so erhaltenen Verbindungen der Formel (I) mit einer Säure umsetzt, um hieraus das Salze zu bilden.

2. Verfahren gemäß Anspruch 1, dadurch gekennzeichnet, daß

wenn in der Formel (III) R ein Wasserstoffatom bedeutet, man ein Produkt der Formel (II) verwendet, worin Hal ein Fluor- oder Bromatom bedeutet;

man gegebenenfalls ein Hydroxylaminsalz, z.B. das Hydrochlorid, mit der Verbindung Ar—Hal in Lösung in einem Alkohol oder einem wäßrig -alkoholischen Milieu und in Gegenwart einer starken Base, wie Natronlauge, Kalilauge, oder eines Alkoholats umsetzt;

man gegebenenfalls die Verbindung der Formel (III), worin die Reste R einen Phthalimidorest bilden, mit einer starken Base, wie Natrium- oder Kaliumhydrid, und danach mit der Verbindung der Formel (II) oder auch direkt mit der Verbindung der Formel (II) in dem Medium eines inerten Lösungsmittels, wie z.B. Acetonitril, in Gegenwart einer tertiären Base, wie Triethylamin oder Pyridin, umsetzt.

3. Verfahren zur Herstellung der Verbindungen der Formel (I) und von deren Salzen gemäß Anspruch 1, dadurch gekennzeichnet, daß man eine Verbindung der Formel

$$Ar—OH \quad (V)$$

worin Ar die vorstehend angegebene Bedeutung besitzt, der Einwirkung einer Verbindung der Formel

$$H_2NOSO_3H \quad (VI)$$

unterzieht, um die entsprechende Verbindung der Formel (I) zu erhalten, die man gewünschtenfalls mit einer Säure umsetzt, um hieraus das Salz zu bilden.

4. Verfahren gemäß einem der Ansprüche 1 bis 3 zur Herstellung der Verbindungen der Formel (I) gemäß Anspruch 1 entsprechend der Formel ($I_A$)

$$Ar'—O—NH_2 \quad (I_A)$$

worin Ar' einen mono- oder polycyclischen, aromatischen oder heteroaromatischen Rest bedeutet, der gegebenenfalls substituiert ist durch einen oder mehrere Reste, ausgewählt unter der OH-Gruppe, den Halogenatomen, den $NO_2$- und CN-Gruppen, den Resten

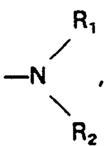 , 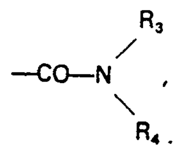 , 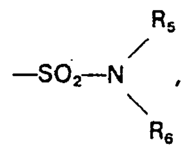 ,

worin $R_1$, $R_2$, $R_3$, $R_4$, $R_5$ und $R_6$, die gleich oder verschieden sind, ein Wasserstoffatom oder einen linearen oder verzweigten Alkylrest mit 1 bis 8 Kohlenstoffatomen bedeuten; den Resten $R_7$ und $OR_8$, wobei $R_7$ und $R_8$ gesättigte oder ungesättigte, lineare oder verzweigte Alkylreste mit bis zu 8 Kohlenstoffatomen, die gegebenenfalls durch ein oder mehrere Halogenatome oder durch eine Cyanogruppe substituiert sind, bedeuten, den Resten $—CO—R_9$, den Resten

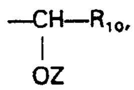

worin Z ein Wasserstoffatom, eine gesättigte oder ungesättigte, lineare oder verzweigte Alkylgruppe mit bis zu 8 Kohlenstoffatomen oder einen Acylrest mit 2 bis 18 Kohlenstoffatomen bedeutet, den Resten $NHCOR_{11}$, $SO_2—R_{12}$, $SO_3—R_{13}$, worin $R_9$, $R_{10}$, $R_{11}$, $R_{12}$ und $R_{13}$ einen Alkylrest mit 1 bis 8 Kohlenstoffatomen bedeuten, den Resten $CO_2—R_{14}$, worin $R_{14}$ ein Wasserstoffatom oder einen Alkylrest mit 1 bis 8 Kohlenstoffatomen bedeutet, und den Arylresten mit 6 bis 14 Kohlenstoffatomen, wobei die Substituenten von Ar' gegebenenfalls Ringe bilden können, die gegebenenfalls ein oder mehrere Heteroatome, ausgewählt unter Sauerstoff, Stickstoff und Schwefel, enthalten, sowie von deren Additionssalze mit Säuren, unter der Voraussetzung, daß Ar' weder einen Phenylrest, der unsubstituiert ist, noch einen Phenylrest, der durch einen Methylrest in 2-, 3- oder 4-Stellung, durch eine Nitrogruppe in 2- oder 4-Stellung, durch ein Chloratom in 3- oder 4-Stellung, durch ein Bromatom in 4-Stellung oder durch eine $CF_3$-Gruppe in 3- oder 4-Stellung monosubstituiert ist, noch einen Phenylrest, der entweder in 2-Stellung durch eine Nitrogruppe und in 4-Stellung durch eine $CF_3$-Gruppe oder durch eine Nitrogruppe disubstituiert ist oder in 2- und 6-Stellung durch eine Nitrogruppe disubstituiert ist, noch einen Phenylrest, der durch Nitrogruppen in 2-, 4- und 6-Stellung oder durch Nitrogruppen in 2- und 4-Stellung und durch eine $CF_3$-Gruppe in 6-Stellung trisubstituiert ist, bedeutet, dadurch gekennzeichnet, daß man von einer Verbindung der Formel (II) oder (V) ausgeht, worin Ar die vorstehenden Definitionen von Ar' besitzt.

5. Verfahren gemäß Anspruch 4, dadurch gekennzeichnet, daß man von einer Verbindung der Formel (II) oder (V) ausgeht, worin Ar einen substituierten Phenylrest bedeutet.

6. Verfahren gemäß Anspruch 4 oder 5, dadurch gekennzeichnet, daß man von einer Verbindung der Formel (II) oder (V) ausgeht, worin Ar einen zumindest durch eine Nitrogruppe substituierten Phenylrest bedeutet.

7. Verfahren gemäß Anspruch 4 oder 5, dadurch gekennzeichnet, daß man von einer Verbindung der Formel (II) oder (V) ausgeht, worin Ar einen Phenylrest bedeutet, der zumindest durch ein Chloratom in para-Stellung substituiert ist.

8. Verfahren gemäß Anspruch 5, 6 oder 7, dadurch gekennzeichnet, daß man von einer Verbindung der Formel (II) oder (V) ausgeht, worin Ar einen Phenylrest bedeutet, der in 2-Stellung durch eine Nitrogruppe und in 4-Stellung durch ein Chloratom substituiert ist.

9. Verfahren gemäß Anspruch 5 oder 6, dadurch gekennzeichnet, daß man von einer Verbindung der Formel (II) oder (V) ausgeht, worin Ar einen Phenylrest bedeutet, der in 4-Stellung durch eine Nitrogruppe und in 3-Stellung durch eine Alkoxygruppe mit 1 bis 8 Kohlenstoffatomen substituiert ist.

10. Verfahren gemäß Anspruch 5 oder 6, dadurch gekennzeichnet, daß man von einer Verbindung der Formel (II) oder (V) ausgeht, worin Ar einen Phenylrest bedeutet, der in 4-Stellung durch eine Nitrogruppe und in 2-Stellung durch eine lineare oder verzweigte Alkylgruppe mit 1 bis 8 Kohlenstoffatomen substituiert ist.

11. Verfahren gemäß Anspruch 4, dadurch gekennzeichnet, daß man von einer Verbindung der Formel (II) oder (V) ausgeht, worin Ar einen Rest

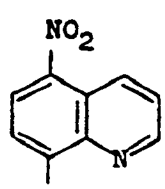

bedeutet, der gegebenenfalls substituiert ist.

12. Verfahren gemäß Anspruch 4, dadurch gekennzeichnet, daß man von einer Verbindung der Formel (II) oder (V) ausgeht, worin Ar einen Rest

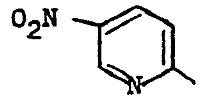

bedeutet.

13. Verfahren gemäß Anspruch 4, dadurch gekennzeichnet, daß man von einer Verbindung der Formel (II) oder (V) ausgeht, worin Ar einen 2-Methyl-4-nitrophenylrest oder einen 3-Methoxy-4-nitrophenylrest bedeutet.

14. Verfahren gemäß Anspruch 4, dadurch gekennzeichnet, daß man von einer Verbindung der Formel (II) oder (V) ausgeht, worin Ar einen Rest bedeutet, ausgewählt unter den Resten
5-Nitro-8-chinolyl;
2-Chlor-4-nitrophenyl;
2-Nitro-4-chlorphenyl;
2,4-Dichlorphenyl;
4-Nitro-2-trifluormethylphenyl;
2-(1-(RS)-Hydroxyethyl)-4-nitrophenyl;
4-Cyanophenyl;
3-(Propin-2-yloxy)-4-nitrophenyl und
3,4-Dichlorphenyl.

15. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung der Formel (I) gemäß Anspruch 1 sowie deren Additionssalze mit Säuren enthalten.

16. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung der Formel (I) gemäß einem der Ansprüche 4 bis 12 sowie deren Additionssalze mit Säuren enthalten.

17. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung gemäß Anspruch 13 enthalten.

18. Zusammensetzungen für die Anwendung in der Landwirtschaft zur Verwendung als Wachstumsfaktoren für Pflanzen, dadurch gekennzeichnet, daß sie als Wirkstoff zumindest eine Verbindung gemäß Anspruch 14 enthalten.

**Claims for the Contracting States: BE CH DE FR GB IT LI LU NL SE**

1. Compounds with the formula (I):

$$Ar—O—NH_2 \qquad (I)$$

in which Ar represents an aromatic or hetero-aromatic, mono- or polycyclic radical, possibly substituted by one or more radicals chosen from the group composed of the OH radical, halogen atoms, $NO_2$ and CN groups,

$$-N\begin{matrix}/R_1\\ \backslash R_2\end{matrix}, \quad -CO-N\begin{matrix}/R_3\\ \backslash R_4\end{matrix}, \quad -SO_2-N\begin{matrix}/R_5\\ \backslash R_6\end{matrix},$$

radicals, in which $R_1$, $R_2$, $R_3$, $R_4$, $R_5$ and $R_6$, being identical or different, each represent a hydrogen atom or a linear or branched alkyl radical containing from 1 to 8 carbon atoms; $R_7$ and $OR_8$ radicals, $R_7$ and $R_8$ representing linear or branched, saturated or unsaturated alkyl radicals containing up to 8 carbon atoms, possibly substituted by one or more halogen atoms or by a cyano radical, $—COR_9$ radicals,

$$\begin{matrix}—CHR_{10},\\ |\\ OZ\end{matrix}$$

radicals, Z representing a hydrogen atom, a linear or branched, saturated or unsaturated alkyl radical, containing up to 8 carbon atoms or an acyl radical containing from 2 to 18 carbon atoms, $NHCOR_{11}$, $SO_2R_{12}$, $SO_3R_{13}$ radicals in which $R_9$, $R_{10}$, $R_{11}$, $R_{12}$ and $R_{13}$ represent an alkyl radical containing from 1 to 8 carbon

atoms, $CO_2R_{14}$ radicals in which $R_{14}$ represents a hydrogen atom or an alkyl radical containing from 1 to 8 carbon atoms, aryl radicals containing from 6 to 14 carbon atoms, $—CH_2—CN$ or $—CH_2—SO_2—R_{15}$ groups in which $R_{15}$ represents an alkyl radical containing from 1 to 8 carbon atoms or an aryl radical containing from 6 to 14 carbon atoms possibly substituted by an alkyl radical containing from 1 to 8 carbon atoms and $—OR_{16}$ groups in which $R_{16}$ represents an aryl radical containing from 6 to 14 carbon atoms, possibly substituted by an alkyl radical containing from 1 to 8 carbon atoms, by a halogen atom or a $NO_2$ radical, the substituents of Ar possibly being able to form rings possibly containing one or more heteroatoms chosen from oxygen, nitrogen and sulphur, as well as their salts of addition with acids, providing that Ar represents neither an unsubstituted phenyl radical, nor a phenyl radical monosubstituted by a methyl radical at 2, 3 or 4, by a nitro radical at 2 or 4, by a chlorine atom at 3 or 4, by a bromine atom at 4 or by a $CF_3$ radical at 3 or 4, nor a phenyl radical either disubstituted at 2 by a nitro radical and at 4 by a $CF_3$ radical or by a nitro radical, or disubstituted at 2 and 6 by a nitro radical nor a phenyl radical trisubstituted by nitro radicals at 2, 4 and 6 or by nitro radicals at 2 and 4 and by a $CF_3$ radical at 6.

2. Compounds with the formula (I) as defined in claim 1, for which Ar represents a substituted phenyl radical, as well as their salts of addition with acids.

3. Compounds with the formula (I) as defined in claim 2 in which at least one of the substituents of the phenyl radical is a nitro radical.

4. Compounds with the formula (I) as defined in claim 2 in which at least one of the substituents of the phenyl radical is a chlorine atom in para.

5. Compounds with the formula (I) as defined in claims 3 and 4 for which Ar represents a phenyl radical substituted at 2 by a nitro radical and at 4 by a chlorine atom.

6. Compounds with the formula (I) as defined in claim 3 for which Ar represents a phenyl radical substituted at 4 by a nitro radical and at 3 by an alkoxyl radical containing from 1 to 8 carbon atoms.

7. Compounds with the formula (I) as defined in claim 3 for which Ar represents a phenyl radical substituted at 4 by a nitro radical and at 2 by a linear or branched alkyl radical containing from 1 to 8 carbon atoms.

8. Compounds with the formula (I) as defined in claim 1 for which Ar represents the radical

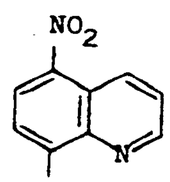

possibly substituted by one or more of the substituents mentioned in claim 1.

9. Compounds with the formula (I) as defined in claim 1, for which Ar represents the radical:

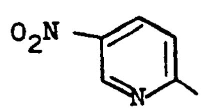

10. Any one of the compounds with the formula (I) as defined in claim 1 the names of which follow:
— O-(2-methyl-4-nitrophenyl)-hydroxylamine,
— O-(4-nitro-3-methoxyphenyl)-hydroxylamine,
as well as their salts of addition with acids.

11. Any one of the compounds with the formula (I) as defined in claim 1, the names of which follow:
— O-(5-nitro-8-quinolyl)-hydroxylamine,
— O-(2-chloro-4-nitrophenyl)-hydroxylamine,
— O-(2-nitro-4-chlorophenyl)-hydroxylamine,
— O-(2,4-dichlorophenyl)-hydroxylamine,
— O-(4-nitro-2-trifluoromethylphenyl)-hydroxylamine,
— O-[2-(1RS-hydroxyethyl)-4-nitrophenyl]-hydroxylamine,
— O-(4-cyanophenyl)-hydroxylamine,
— O-[3-(propyn-2-yloxy)-4-nitrophenyl]-hydroxylamine,
— O-(3,4-dichlorophenyl)-hydroxylamine,
as well as their salts of addition with acids.

12. Compounds with the formula (I), as defined in any one of the claims 1 to 11, as well as their salts of addition with acids, for their use as growth factors for vegetation.

13. Compositions intended for agricultural use as growth factors for vegetation, characterized in that they contain as active principle at least one compound with the formula (I) as defined in any one of the claims 1 to 9, as well as their salts of addition with acids.

14. Compositions intended for agricultural use as growth factors for vegetation, characterized in that they contain as active principle at least one compound defined in claim 10.

15. Compositions intended for agricultural use as growth factors for vegetation, characterized in that they contain as active principle at least one compound defined in claim 11.

16. Preparation process for the compounds with the formula (I) and their salts, as defined in any one of the claims 1 to 11, characterized in that a compound with the formula:

$$Ar—Hal \qquad (II)$$

in which Ar retains the same significance as previously and Hal represents a halogen atom, is submitted to the action of a compound with the formula:

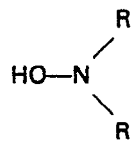

(III)

in which R represents a hydrogen atom or the two radicals R form a phthalimido residue, so as to obtain a compound with the formula:

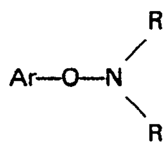

(IV)

in which Ar and R have the significance already given, corresponding to a compound with the formula (I), when R represents a hydrogen atom, which compound with the formula (IV) is made to react, when R is not hydrogen, with hydrazine, so as to obtain a compound with the formula (I), then if desired, the compounds with the formula (I) obtained are made to react with an acid, so as to form their salts.

17. Preparation process for the compounds with the formula (I) and their salts, as defined in any one of the claims 1 to 11, characterized in that a compound with the formula:

$$Ar—OH \qquad (V)$$

in which Ar retains its preceding significance, is submitted to the action of a compound with the formula:

$$H_2NOSO_3H \qquad (VI)$$

so as to obtain the corresponding compound with the formula (I), which, if desired, is made to react with an acid so as to form its salt.

**Claims for the Contracting State: AT**

1. Preparation process for compounds with the formula (I):

$$Ar—O—NH_2 \qquad (I)$$

in which Ar represents an aromatic or hetero-aromatic, mono- or polycyclic radical, possibly substituted by one or more radicals chosen from the group composed of the OH radical, halogen atoms, $NO_2$ and CN groups,

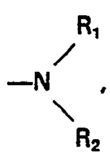 , 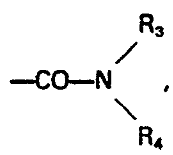 , 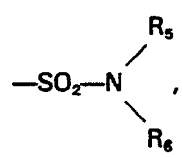 ,

radicals, in which $R_1$, $R_2$, $R_3$, $R_4$, $R_5$ and $R_6$, being identical or different, each represent a hydrogen atom or a linear or branched alkyl radical containing from 1 to 8 carbon atoms; $R_7$ and $OR_8$ radicals, $R_7$ and $R_8$ representing linear or branched, saturated or unsaturated alkyl radicals containing up to 8 carbon atoms, possibly substituted by one or more halogen atoms or by a cyano radical, $—COR_9$ radicals,

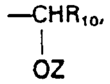

radicals, Z representing a hydrogen atom, a linear or branched, saturated or unsaturated alkyl radical, containing up to 8 carbon atoms or an acyl radical containing from 2 to 18 carbon atoms, $NHCOR_{11}$, $SO_2R_{12}$, $SO_3R_{13}$ radicals in which $R_9$, $R_{10}$, $R_{11}$, $R_{12}$ and $R_{13}$ represent an alkyl radical containing from 1 to 8 carbon atoms, $CO_2R_{14}$ radicals in which $R_{14}$ represents a hydrogen atom or an alkyl radical containing from 1 to 8 carbon atoms, aryl radicals containing from 6 to 14 carbon atoms, $—CH_2—CN$ or $—CH_2—SO_2—R_{15}$ groups in which $R_{15}$ represents an alkyl radical containing from 1 to 8 carbon atoms or an aryl radical containing from 6 to 14 carbon atoms possibly substituted by an alkyl radical containing from 1 to 8 carbon atoms and $—OR_{16}$ groups in which $R_{16}$ represents an aryl radical containing from 6 to 14 carbon atoms, possibly substituted by an alkyl radical containing from 1 to 8 carbon atoms, by a halogen atom or a $NO_2$ radical, the substituents of Ar possibly being able to form rings possibly containing one or more heteroatoms chosen from oxygen, nitrogen and sulphur, as well as their salts of addition with acids, providing that Ar represents neither an unsubstituted phenyl radical, nor a phenyl radical monosubstituted by a methyl radical at 2, 3 or 4, by a nitro radical at 2 or 4, by a chlorine atom at 3 or 4, by a bromine atom at 4 or by a $CF_3$ radical at 3 or 4, nor a phenyl radical either disubstituted at 2 by a nitro radical and at 4 by a $CF_3$ radical or by a nitro radical, or disubstituted at 2 and 6 by a nitro radical, nor a phenyl radical trisubstituted by nitro radicals at 2, 4 and 6 or by nitro radicals at 2 and 4 and by a $CF_3$ radical at 6, characterized in that a compound with the formula:

$$Ar—Hal \qquad (II)$$

in which Ar retains the same significance as previously and Hal represents a halogen atom, is submitted to the action of a compound with the formula:

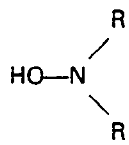

(III)

in which R represents a hydrogen atom or the two radicals R form a phthalimido residue, so as to obtain a compound with the formula:

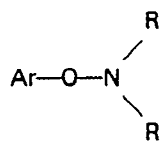

(IV)

in which Ar and R have the significance already given, corresponding to a compound with the formula (I), when R represents a hydrogen atom, which compound with the formula (IV) is made to react, when R is not hydrogen, with hydrazine, so as to obtain a compound with the formula (I), then if desired, the compounds with the formula (I) obtained are made to react with an acid, so as to form their salts.

2. Process according to claim 1, characterized in that

— when, in the formula (III), R represents a hydrogen atom, a product with the formula (II) is used in which Hal represents a fluorine or bromine atom;

— if applicable, a salt of hydroxylamine, for example, hydrochloride, is made to react on the compound Ar—Hal in solution in an alcohol or in a hydroalcohol medium and in the presence of a strong base such as sodium hydroxide, potassium hydroxide or an alcoholate;

— if applicable, the compound with the formula (III) in which the radicals R form a phthalimido residue, is made to react with a strong base such as sodium hydride or potassium hydride, then with the compound with the formula (II), or, also, directly with the compound with the formula (II) in an inert solvent such as, for example, acetonitrile in the presence of a tertiary base such as triethylamine or pyridine.

3. Preparation process for compounds with the formula (I) and their salts, as defined in claim 1, characterized in that a compound with the formula:

$$Ar—OH \qquad (V)$$

in which Ar retains its preceding significance, is submitted to the action of a compound with the formula:

$$H_2NOSO_3H \qquad (VI)$$

so as to obtain the corresponding compound with the formula (I), which, if desired, is made to react with an acid so as to form its salt.

4. Process according to any one of the claims 1 to 3, for the preparation of compounds with the formula (I), as defined in claim 1, answering to the formula ($I_A$):

$$Ar'—O—NH_2 \qquad (I_A)$$

in which Ar' represents an aromatic or hetero-aromatic, mono- or polycyclic radical, possibly substituted by one or more radicals chosen from the group composed of the OH radical, halogen atoms, $NO_2$ and CN groups,

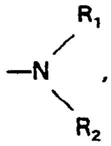 , 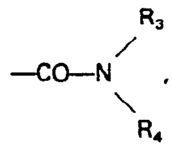 , 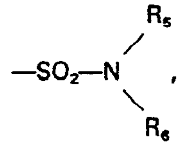 ,

radicals, in which $R_1$, $R_2$, $R_3$, $R_4$, $R_5$ and $R_6$, being identical or different, each represent a hydrogen atom or a linear or branched alkyl radical containing from 1 to 8 carbon atoms; $R_7$ and $OR_8$ radicals, $R_7$ and $R_8$ representing linear or branched, saturated or unsaturated alkyl radicals containing up to 8 carbon atoms, possibly substituted by one or more halogen atoms or by a cyano radical, $—COR_9$ radicals,

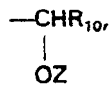

radicals, Z representing a hydrogen atom, a linear or branched, saturated or unsaturated alkyl radical, containing up to 8 carbon atoms or an acyl radical containing from 2 to 18 carbon atoms, $NHCOR_{11}$, $SO_2R_{12}$, $SO_3R_{13}$ radicals in which $R_9$, $R_{10}$, $R_{11}$, $R_{12}$ and $R_{13}$ represent an alkyl radical containing from 1 to 8 carbon atoms, $CO_2R_{14}$ radicals in which $R_{14}$ represents a hydrogen atom or an alkyl radical containing from 1 to 8 carbon atoms, and aryl radicals containing from 6 to 14 carbon atoms, the substituents of Ar' possibly being able to form rings possibly containing one or more heteroatoms chosen from oxygen, nitrogen and sulphur, as well as their salts of addition with acids, providing that Ar' represents neither an unsubstituted phenyl radical, nor a phenyl radical monosubstituted by a methyl radical at 2, 3 or 4, by a nitro radical at 2 or 4, by a chlorine atom at 3 or 4, by a bromine atom at 4 or by a $CF_3$ radical at 3 or 4, nor a phenyl radical either disubstituted at 2 by a nitro radical and at 4 by a $CF_3$ radical or by a nitro radical, or disubstituted at 2 and 6 by a nitro radical, nor a phenyl radical trisubstituted by nitro radicals at 2, 4 and 6 or by nitro radicals at 2 and 4 and by a $CF_3$ radical at 6, characterized in that at the start a compound with the formula (II) or (V) is used in which Ar has the significances of Ar' above.

5. Process according to claim 4, characterized in that at the start a compound with the formula (II) or (V) is used in which Ar represents a substituted phenyl radical.

6. Process according to claim 4 or 5, characterized in that at the start a compound with the formula (II) or (V) is used in which Ar represents a phenyl radical substituted by at least one nitro radical.

7. Process according to claim 4 or 5, characterized in that at the start a compound with the formula (II) or (V) is used in which Ar represents a phenyl radical substituted by at least one chlorine atom in para position.

8. Process according to claim 5, 6 or 7, characterized in that at the start a compound with the formula (II) or (V) is used in which Ar represents a phenyl radical substituted at 2 by a nitro radical and at 4 by a chlorine atom.

9. Process according to claim 5 or 6, characterized in that at the start a compound with the formula (II) or (V) is used in which Ar represents a phenyl radical substituted at 4 by a nitro radical and at 3 by an alkoxyl radical containing from 1 to 8 carbon atoms.

10. Process according to claim 5 or 6, characterized in that at the start a compound with the formula (II) or (V) is used in which Ar represents a phenyl radical substituted at 4 by a nitro radical and at 2 by a linear or branched alkyl radical containing from 1 to 8 carbon atoms.

11. Process according to claim 4, characterized in that at the start a compound with the formula (II) or (V) is used in which Ar represents a possibly substituted radical:

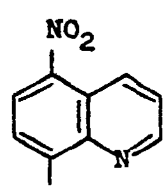

12. Process according to claim 4, characterized in that at the start a compound with the formula (II) or (V) is used in which Ar represents a radical:

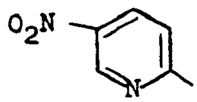

13. Process according to claim 4, characterized in that at the start a compound with the formula (II) or (V) is used in which Ar represents a 2-methyl-4-nitrophenyl or a 3-methoxy-4-nitrophenyl radical.

14. Process according to claim 4, characterized in that at the start a compound with the formula (II) or (V) is used in which Ar represents a radical chosen from the group formed by the radicals:

— 5-nitro-8-quinolyl;
— 2-chloro-4-nitrophenyl;
— 2-nitro-4-chlorophenyl;
— 2,4-dichlorophenyl;
— 4-nitro-2-trifluoromethylphenyl;
— 2-(1-(RS)-hydroxyethyl)-4-nitrophenyl;
— 4-cyanophenyl;
— 3-(propyn-2-yloxy)-4-nitrophenyl and
— 3,4-dichlorophenyl.

15. Compositions intended for agricultural use as growth factors for vegetation, characterized in that they contain as active principle at least one compound with the formula (I) as defined in claims 1, as well as their salts of addition with acids.

16. Compositions intended for agricultural use as growth factors for vegetation, characterized in that they contain as active principle at least one compound with the formula (I), as defined in any one of the claims 4 to 12, as well as their salts of addition with acids.

17. Compositions intended for agricultural use as growth factors for vegetation, characterized in that they contain as active principle at least one compound defined in claim 13.

16. Compositions intended for agricultural use as growth factors for vegetation, characterized in that they contain as active principle at least one compound defined in claim 14.